# EUROPEAN PATENT APPLICATION

(11) **EP 4 494 695 A1**
(43) Date of publication of application: **22.01.2025**
(21) Application number: 23770905.0
(22) Date of filing: 17.03.2023
(51) Int. Cl.: A61P 1/00, A61P 1/04, A61P 1/12, A61P 1/14, A61P 1/16, A61P 3/00, A61P 3/04, A61P 3/06, A61P 3/10, A61P 9/04, A61P 9/06, A61P 11/06, A61P 13/12, A61P 31/04, A61P 35/00, A61P 37/08, A61P 43/00, C07H 3/02, C07H 3/04, C07H 3/06, C07H 3/08

(54) **COMPOSITION FOR CONTROLLING PROLIFERATION OF BACTERIUM IN INTESTINE, AND USE THEREOF**

(30) Priority: 18.03.2022 JP 2022044397; 18.03.2022 JP 2022044398; 18.03.2022 JP 2022044399; 18.03.2022 JP 2022044400; 18.03.2022 JP 2022044401; 18.03.2022 JP 2022044402; 18.03.2022 JP 2022044403; 18.03.2022 JP 2022044404; 18.03.2022 JP 2022044405; 18.03.2022 JP 2022044406; 18.03.2022 JP 2022044407
(71) Applicant: MEIJI CO., LTD, Chuo-ku Tokyo 104-8306 (JP)
(72) Inventor: FUJIWARA Shin, Hachioji-shi, Tokyo 192-0919 (JP); TAKEDA Mariko, Hachioji-shi, Tokyo 192-0919 (JP)
(74) Representative: TBK
(86) International application number: PCT/JP2023/010498
(87) International publication number: WO 2023/176952

(57) **Abstract**

The object of the invention is to provide a means for controlling growth of bacteria in the intestinal tract. There is provided a composition for controlling growth of any bacteria selected from the group consisting bacteria of the genus *Eggerthella,* bacteria of the genus *Oscillibacter*, bacteria of the genus *Holdemania*, and bacteria of the genus *Parasutterella* in the intestinal bacterial microbiota, which contains any selected from the group consisting of a human milk oligosaccharide and a constituent sugar of a human milk oligosaccharide.

## Description

### Technical Field

The present invention relates to a composition for controlling growth of bacteria in the intestinal tract and use thereof.

### Background Art

In recent years, it has become clear that intestinal bacteria affect a variety of physiological functions in the host, suggesting a link between intestinal bacteria and mental and behavioral disorders, including mood disorder such as depression.

It has been reported that when feces from depressed patients and normal subjects were administered to germ-free mice, depression-like behaviors increased in mice administered feces from depressed patients compared with mice administered feces from normal subjects (Non-patent document 1). In addition, a systematic review comparing the intestinal bacteria of healthy and depressed individuals reported that intestinal bacteria of 24 genera in total, including bacteria of the genera *Eggerthella, Oscillibacter, Holdemania,* and *Parasutterella,* were more abundant in depressed individuals (Non-patent document 2).

Antibiotics such as metronidazole and vancomycin are known as means for decreasing bacteria of the genus *Eggerthella* (Non-patent document 3). It has also been reported that administration of *Lactobaccilus plantarum* CCFM1143 for 4 weeks to patients with chronic diarrhea relieved clinical symptoms and regulated the intestinal bacterial microbiota, particularly decreasing the abundance of bacteria of the genera *Bacteroides* and *Eggerthella* (Non-patent document 4). Furthermore, Patent document 1 describes an agent for suppressing the number of *Eggerthella* bacteria, which contains 1-kestose, a type of oligosaccharide also found in vegetables and grains such as onion, garlic, barley, and rye, as the active ingredient, and describes that this agent can be used for the prevention or treatment of type 2 diabetes and diseases selected from bacteremia, sinusitis, pyogenic myositis, skin abscess, spinal discitis, and liver abscess caused by bacteria of the genus *Eggerthella.*

With regard to bacteria of the genus *Oscillibacter,* it has been reported that pretreatment of rats with acute heart failure with *Bifidobacterium longum* R0175 reduced the relative amounts of potentially harmful bacteria such as bacteria of the genus *Oscillibacter* and partially restored the abnormal intestinal bacterial microbiota in rats with liver injury (Non-patent document 5). It has also been reported that isoorientin inhibited growth of bacteria of the genus *Oscillibacter* which can cause inflammation in the intestinal microbiota in mice (Non-patent document 6). Furthermore, it has also been reported that administration of nicotinamide mononucleotide (NMN) to mice increased butyrate-producing bacteria and decreased several harmful bacteria including bacteria of the genus *Oscillibacter* in their feces (Non-patent document 7). In addition, Patent document 2 describes that a herbal medicine, natural product, medicinal herb or composition containing a medicinal herb selectively increase the first intestinal bacterial microbiota population in the subject, while simultaneously decreasing the second intestinal bacterial microbiota population. This reference further describes that the first intestinal bacterial microbiota population includes short-chain fatty acid (SCFA)-producing bacteria, and mentions *Oscillibacter* bacteria as one class of the bacteria of the first intestinal bacterial microbiota population. Further, Patent document 3 describes a composition for fecal microbiota transplantation to prevent or reduce inflammation induced by systemic and intestinal therapies, and mentions *Oscillibacter* bacteria as one class of bacteria that increase after fecal transplantation. In addition, Patent document 4 describes that soluble corn fibers increase bacteria of the genus *Oscillibacter* and other bacteria.

With regard to bacteria of the genus *Holdemania,* it has been reported that in the intestinal bacterial microbiota of healthy individuals who orally consumed a probiotic product containing *Bifidobacterium longum* BB536 and *Lactobacillus rhamnosus* HN001 for one month, decrease of bacteria such as *Holdemania filiformis* was observed at species level (Non-patent document 8). In addition, Patent document 2 mentioned above describes *Holdemania* bacteria as one class of the bacteria included in the first intestinal bacterial microbiota population.

With respect to bacteria of the genus *Parasutterella,* it has been reported that occupancy rate thereof is greater in patients suffering from major depressive and depressive disorders (Non-patent documents 9 and 10). It has also been reported that, in an experiment aiming at evaluating the effect of inulin and metformin for suppressing polycystic ovary syndrome (PCOS) in mice and the related mechanisms, inulin and metformin improved the morphology of PCOS, and the results of sequence analysis of the intestinal bacterial microbiota showed that inulin intake group had increased *Bifidobacterium* bacteria and decreased *Proteobacteria, Helicobacter,* and *Parasutterella* bacteria (Non-patent document 11).

By the way, human milk oligosaccharides (HMOs) are the third most abundant solid component in breast milk after lactose and lipids. It is known that feeding 2'-fucosyllactose, 3-fucosyllactose, and lactodifucotetraose, types of human milk oligosaccharides, causes bifidobacteria to grow (Non-patent document 12).

### Prior Art References

### Patent documents

Patent document 1: Japanese Patent Publication (Kokai) No. 2020-143020
Patent document 2: WO2013/182038 (Japanese Patent Publication (Kohyo) No. 2015-520176)
Patent document 3: WO2020/016445 (Japanese Patent Publication (Kohyo) No. 2021-530527)
Patent document 4: Japanese Patent Publication (Kokai) No. 2020-120675

### Non-patent documents

Non-patent document 1: Gut microbiome remodeling induces depressive-like behaviors through a pathway mediated by the host's metabolism. Mol Psychiatry. 2016;21(6):786-796. doi:10.1038/mp.2016.44
Non-patent document 2: Altered Composition of Gut Microbiota in Depression: A Systematic Review. Front Psychiatry. 2020; 11:541. Published 2020 Jun 10. doi: 10.3389/fpsyt.2020.00541
Non-patent document 3: Bo J, Wang S, Bi Y, Ma S, Wang M, Du Z. Eggerthella lenta bloodstream infections: two cases and review of the literature [published correction appears in Future Microbiol. 2020 Nov;15:1689]. Future Microbiol. 2020;15:981-985. doi:10.2217/fmb-2019-0338
Non-patent document 4: Yang B, Yue Y, Chen Y, et al. Lactobacillus plantarum CCFM1143 Alleviates Chronic Diarrhea via Inflammation Regulation and Gut Microbiota Modulation: A Double-Blind, Randomized, Placebo-Controlled Study. Front Immunol. 2021;12:746585. Published 2021 Oct 15. doi:10.3389/fimmu.2021.746585
Non-patent document 5: Wang K, Lv L, Yan R, et al. Bifidobacterium longum R0175 Protects Rats against d-Galactosamine-Induced Acute Liver Failure. mSphere. 2020;5(1):e00791-19. Published 2020 Jan 29. doi:10.1128/mSphere.00791-19
Non-patent document 6: Yuan L, Li X, He S, Gao C, Wang C, Shao Y Effects of Natural Flavonoid Isoorientin on Growth Performance and Gut Microbiota of Mice. J Agric Food Chem. 2018;66(37):9777-9784. doi:10.1021/acs.jafc.8b03568
Non-patent document 7: Huang P, Jiang A, Wang X, et al. NMN Maintains Intestinal Homeostasis by Regulating the Gut Microbiota. Front Nutr. 2021;8:714604. Published 2021 Jul 29. doi:10.3389/fnut.2021.714604
Non-patent document 8 Toscano M, De Grandi R, Stronati L, De Vecchi E, Drago L. Effect of Lactobacillus rhamnosus HN001 and Bifidobacterium longum BB536 on the healthy gut microbiota composition at phyla and species level: A preliminary study. World J Gastroenterol. 2017;23(15):2696-2704. doi:10.3748/wjg.v23.i15.2696
Non-patent document 9: Cheung SG, Goldenthal AR, Uhlemann AC, Mann JJ, Miller JM, Sublette ME. Systematic Review of Gut Microbiota and Major Depression. Front Psychiatry. 2019;10:34. Published 2019 Feb 11. doi:10.3389/fpsyt.2019.00034
Non-patent document 10: Barandouzi ZA, Starkweather AR, Henderson WA, Gyamfi A, Cong XS. Altered Composition of Gut Microbiota in Depression: A Systematic Review. Front Psychiatry. 2020;11:541. Published 2020 Jun 10. doi:10.3389/fpsyt.2020.00541
Non-patent document 11: Xue J, Li X, Liu P, et al. Inulin and metformin ameliorate polycystic ovary syndrome via anti-inflammation and modulating gut microbiota in mice. Endocr J. 2019;66(10):859-870. doi:10.1507/endocj.EJ18-0567
Non-patent document 12: Yu ZT, Chen C, Newburg DS. Utilization of major fucosylated and sialylated human milk oligosaccharides by isolated human gut microbes. Glycobiology. 2013;23(11):1281-1292. doi:10.1093/glycob/cwt065

### Summary of the Invention

### Object to be achieved by the invention

Any new material that can control growth of bacteria in the intestinal tract known to be associated with mental and behavioral disorders, including mood disorder such as depression, would be desirable.

### Means for achieving the object

The present invention provides the followings
[1] A composition for controlling growth of any bacteria selected from the group consisting bacteria of the genus *Eggerthella,* bacteria of the genus *Oscillibacter,* bacteria of the genus *Holdemania,* and bacteria of the genus *Parasutterella* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, lactose, and fucose, and is for controlling growth of bacteria of the genus *Eggerthella.*
[3] The composition according to 2, which contains any selected from the group consisting of 2'-fucosyllactose, 3'-sialyllactose, 6'-sialyllactose, lactose, and fucose.
[4] The composition according to 1, which contains any selected from the group consisting of a fucosylated human milk oligosaccharide, a human milk oligosaccharide containing lactose as a constituent sugar, a human milk oligosaccharide containing fucose as a constituent sugar, lactose, and fucose, and is for controlling growth of any bacteria selected from the group consisting of bacteria of the genus *Eggerthella* and bacteria of the genus *Oscillibacter.*
[5] The composition according to 4, which contains any selected from the group consisting of 2'-fucosyllactose, lactose, and fucose.
[6] The composition according to 1, which contains any selected from the group consisting of a fucosylated human milk oligosaccharide, a sialylated human milk oligosaccharide, a human milk oligosaccharide containing lactose as a component sugar, a human milk oligosaccharide containing sialic acid as a constituent sugar, lactose, and sialic acid, and is for controlling growth of any bacteria selected from the group consisting of bacteria of the genus *Holdemania* and bacteria of the genus *Parasutterella.*
[7] The composition according to 6, which contains any selected from the group consisting of 2'-fucosyllactose, 3'-sialyllactose, 6'-sialyllactose, lactose, and N-acetylneuraminic acid.
[8] The composition according to any one of 1 to 7, which is for use as a prebiotic or synbiotic.
[9] The composition according to any one of 1 to 8, which is for treating any selected from the group consisting of depression and mood disorder.
[10] A composition for treating a disease or condition that can be ameliorated by controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal tract, which contains any selected from the group consisting of a human milk oligosaccharide and a constituent sugar of a human milk oligosaccharide.
[11] A composition for treating any selected from the group consisting of depression and mood disorder, which contains any selected from the group consisting of a human milk oligosaccharide and a constituent sugar of a human milk oligosaccharide.
[12] The composition according to any one of 1 to 10, wherein the control of growth is suppression of growth.
[13] A composition for use in a method for controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal bacterial microbiota, a method or non-therapeutic method for controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal bacterial microbiota, which comprises the step of administering a composition containing any selected from the group consisting of a human milk oligosaccharide and a constituent sugar of a human milk oligosaccharide to a subject, or use or non-therapeutic use of a composition containing any selected from the group consisting of a human milk oligosaccharide and a constituent sugar of a human milk oligosaccharide for controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal bacterial microbiota.
[14] The composition according to 13, which is for use in a method for controlling growth of bacteria of the genus *Eggerthella,* and contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, lactose, and fucose; the use in manufacture according to 13, which is of any selected from a fucosylated human milk oligosaccharide, a sialylated human milk oligosaccharide, a human milk oligosaccharide containing lactose as a constituent sugar, a human milk oligosaccharide containing fucose as a constituent sugar, lactose, and fucose, and is in manufacture of a composition for controlling growth of bacteria of the genus *Eggerthella,* the method or non-therapeutic method according to 13, which comprises the step of administering a composition containing any selected from a fucosylated human milk oligosaccharide, a sialylated human milk oligosaccharide, a human milk oligosaccharide containing lactose as a constituent sugar, a human milk oligosaccharide containing fucose as a constituent sugar, lactose, and fucose to a subject, and is for controlling growth of bacteria of the genus *Eggerthella*; or the use or non-therapeutic use according to 13, which is of such a composition for controlling growth of bacteria of the genus *Eggerthella.*
[15] The composition according to 14, which contains any selected from the group consisting of 2'-fucosyllactose, 3'-sialyllactose, 6'-sialyllactose, lactose, and fucose; the use in manufacture according to 14, which is of any selected from the group consisting of 2'-fucosyllactose, 3'-sialyllactose, 6'-sialyllactose, lactose, and fucose; the method or non-therapeutic method according to 14, which comprises the step of administering a composition containing any selected from the group consisting of 2'-fucosyllactose, 3'-sialyllactose, 6'-sialyllactose, lactose, and fucose to a subject; or the use or non-therapeutic use according to 14, which is of such a composition.
[16] The composition according to 13, which is for use in a method for controlling growth of any bacteria selected from the group consisting of bacteria of the genus *Eggerthella* and bacteria of the genus *Oscillibacter,* and contains any selected from the group consisting of a fucosylated human milk oligosaccharide, a human milk oligosaccharide containing lactose as a constituent sugar, a human milk oligosaccharide containing fucose as a constituent sugar, lactose, and fucose; the use in manufacture according to 13, which is of any selected from the group consisting of a fucosylated human milk oligosaccharide, a human milk oligosaccharide containing lactose as a constituent sugar, a human milk oligosaccharide containing fucose as a constituent sugar, lactose, and fucose, and is in manufacture of a composition for controlling growth of any bacteria selected from the group consisting of bacteria of the genus *Eggerthella* and bacteria of the genus *Oscillibacter*; the method or non-therapeutic method according to 13, which comprises the step of administering a composition containing any selected from the group consisting of a fucosylated human milk oligosaccharide, a human milk oligosaccharide containing lactose as a constituent sugar, a human milk oligosaccharide containing fucose as a constituent sugar, lactose, and fucose to a subject, and is for controlling growth of any bacteria selected from the group consisting of bacteria of the genus *Eggerthella* and bacteria of the genus *Oscillibacter,* or the use or non-therapeutic use according to 13, which is of such a composition for controlling growth of any bacteria selected from the group consisting of bacteria of the genus *Eggerthella* and bacteria of the genus *Oscillibacter.*
[17] The composition according to 16, which contains any selected from the group consisting of 2'-fucosyllactose, lactose, and fucose; the use in manufacture according to 16, which is of any selected from the group consisting of 2'-fucosyllactose, lactose, and fucose; the method or non-therapeutic method according to 16, which comprises the step of administering a composition comprising any selected from the group consisting of 2'-fucosyllactose, lactose, and fucose to a subject; or the use or non-therapeutic use according to 16, which is of such a composition.
[18] A composition for use in a method for controlling growth of any bacteria selected from the group consisting of bacteria of the genus *Holdemania* and bacteria of the genus *Parasutterella,* which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid; use of any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid in manufacture of a composition for controlling growth of any bacteria selected from the group consisting of bacteria of the genus *Holdemania* and bacteria of the genus *Parasutterella*; a method or non-therapeutic method for controlling growth of any bacteria selected from the group consisting of bacteria of the genus *Holdemania* and bacteria of the genus *Parasutterella,* which comprises the step of administering a composition containing any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid to a subject; or use or non-therapeutic use of such a composition for controlling growth of any bacteria selected from the group consisting of bacteria of the genus *Holdemania* and bacteria of the genus *Parasutterella.*
[19] The composition according to 18, which contains any selected from the group consisting of 2'-fucosyllactose, 3'-sialyllactose, 6'-sialyllactose, lactose, and N-acetylneuraminic acid; the use in manufacture according to 18, which is of any selected from the group consisting of 2'-fucosyllactose, 3'-sialyllactose, 6'-sialyllactose, lactose, and N-acetylneuraminic acid; the method or non-therapeutic method according to 18, which comprises the step of administering a composition containing any selected from the group consisting of 2'-fucosyllactose, 3'-sialyllactose, 6'-sialyllactose, lactose, and N-acetylneuraminic acid to a subject; or the use or non-therapeutic use according to 18, which is of such a composition.
[20] The composition, use in manufacture, method or non-therapeutic method, or use or non-therapeutic use according to any one of 13 to 19, wherein the composition is for use as a prebiotic or symbiotic.
[21] The composition according to any one of 13 to 20, which is for use in a method for treating any selected from the group consisting of depression and mood disorder; the use in manufacture according to any one of 13 to 20, which is in manufacture of a composition for treating any selected from the group consisting of depression and mood disorder; the method or non-therapeutic method according to any one of 13 to 20, which is for treating any selected from the group consisting of depression and mood disorder; or the use or non-therapeutic use according to any one of 13 to 20, which is for treating any selected from the group consisting of depression and mood disorder.
[22] A composition for use in a method for treating a disease or condition that can be ameliorated by controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal tract, which contains any selected from the group consisting of a human milk oligosaccharide and a constituent sugar of a human milk oligosaccharide; use of any selected from the group consisting of a human milk oligosaccharide and a constituent sugar of a human milk oligosaccharide in manufacture of a composition for treating a disease or condition that can be ameliorated by controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal tract; a method or non-therapeutic method for treating a disease or condition that can be ameliorated by controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal tract, which comprises the step of administering a composition containing any selected from the group consisting of a human milk oligosaccharide and a constituent sugar of a human milk oligosaccharide to a subject; or use or non-therapeutic use of a composition containing any selected from the group consisting of a human milk oligosaccharide and a constituent sugar of a human milk oligosaccharide for treating a disease or condition that can be ameliorated by controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal tract.
[23] A composition for use in a method for treating any selected from the group consisting of depression and mood disorder, which contains any selected from the group consisting of a human milk oligosaccharide and a constituent sugar of a human milk oligosaccharide; use of any selected from the group consisting of a human milk oligosaccharide and a constituent sugar of a human milk oligosaccharide in manufacture of a composition for treating any selected from the group consisting of depression and mood disorder; a method or non-therapeutic method for treating any selected from the group consisting of depression and mood disorder, which comprises the step of administering a composition containing any selected from the group consisting of a human milk oligosaccharide and a constituent sugar of a human milk oligosaccharide to a subject; or use or non-therapeutic use of a composition containing any selected from the group consisting of a human milk oligosaccharide and a constituent sugar of a human milk oligosaccharide for treating any selected from the group consisting of depression and mood disorder.
[24] The composition, use in manufacture, method or non-therapeutic method, or use or non-therapeutic use according to any one of 13 to 22, wherein the control of growth is suppression of growth.

The present invention provides the followings.
[1] A composition for controlling growth of bacteria of the genus *Eggerthella* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, lactose, and fucose.
[3] The composition according to 1 or 2, which contains any selected from the group consisting of 2'-fucosyllactose, 3'-sialyllactose, 6'-sialyllactose, lactose, and fucose.
[4] The composition according to any one of 1 to 3, which is for use as a prebiotic or synbiotic.
[5] The composition according to any one of 1 to 4, which is for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin.
[6] A composition for treating a disease or condition that can be ameliorated by controlling growth of bacteria of the genus *Eggerthella* in the intestinal tract, which contains any selected from the group consisting of a human milk oligosaccharide and a constituent sugar of a human milk oligosaccharide.
[7] A composition for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin, which contains any selected from the group consisting of a human milk oligosaccharide and a constituent sugar of a human milk oligosaccharide.
[8] The composition according to any one of 1 to 6, wherein the control of growth is suppression of growth.
[9] A composition for use in a method for controlling growth of bacteria of the genus *Eggerthella* in the intestinal bacterial microbiota, which contains any selected from the group consisting of a human milk oligosaccharide and a constituent sugar of a human milk oligosaccharide; use of any selected from the group consisting of a human milk oligosaccharide and a constituent sugar of a human milk oligosaccharide in manufacture of a composition for controlling growth of bacteria of the genus *Eggerthella* in the intestinal bacterial microbiota; a method for controlling growth of bacteria of the genus *Eggerthella* in the intestinal bacterial microbiota, which comprises the step of administering a composition containing any selected from the group consisting of a human milk oligosaccharide and a constituent sugar of a human milk oligosaccharide to a subject; or use of a composition containing any selected from the group consisting of a human milk oligosaccharide and a constituent sugar of a human milk oligosaccharide for controlling growth of bacteria of the genus *Eggerthella* in the intestinal bacterial microbiota.
[10] The composition according to 9, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, lactose, and fucose; or the use in manufacture, method, or use according to 9, wherein the composition is such a composition.
[11] The composition according to 9 or 10, which contains any selected from the group consisting of 2'-fucosyllactose, 3'-sialyllactose, 6'-sialyllactose, lactose, and fucose; or the use in manufacture, method, or use according to 9 or 10, wherein the composition is such a composition.
[12] The composition according to any one of 9 to 11, which is for use as a prebiotic or synbiotic; the use in manufacture according to any one of 9 to 11, wherein the composition is such a composition; the method according to any one of 9 to 11, which comprises the step of administering the composition as a prebiotic or synbiotic; or the use according to any one of 9 to 11, wherein the composition is used as a prebiotic or synbiotic.
[13] The composition according to any one of 9 to 12, which is for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin; the use in manufacture according to any one of 9 to 12, wherein the composition is such a composition; the method according to any one of 9 to 12, which is for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin; or the use according to any one of 9 to 12, which is for such a treatment.
[14] A composition for treating a disease or condition that can be ameliorated by controlling growth of bacteria of the genus *Eggerthella* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Eggerthella* in the intestinal tract, a method for treating a disease or condition that can be ameliorated by controlling growth of bacteria of the genus *Eggerthella* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Eggerthella* in the intestinal tract.
[15] A composition for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin, a method for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of inflammatory bowel disease, multiple sclerosis, rheumatoid arthritis, mood disorder, and asthma, for administration to a patient with colitis aggravated by activation of helper T17 cells, or for administration to a patient with heart failure or arrhythmia who is being administered digoxin.
[16] The composition, use in manufacture of a composition, method, or use according to any one of 9 to 14, wherein the control of growth is suppression of growth.

The present invention provides the followings
[1] A composition for controlling growth of bacteria of the genus *Oscillibacter* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a human milk oligosaccharide containing lactose as a constituent sugar, a human milk oligosaccharide containing fucose as a constituent sugar, lactose, and fucose.
[3] The composition according to 1 or 2, which contains any selected from the group consisting of 2'-fucosyllactose, lactose, and fucose.
[4] The composition according to any one of 1 to 3, which is for use as a prebiotic or synbiotic.
[5] The composition according to any one of 1 to 4, which is for treating any selected from the group consisting of chronic kidney disease, mood disorder, and inflammatory bowel disease.
[6] A composition for treating a disease or condition that can be ameliorated by controlling growth of bacteria of the genus *Oscillibacter* in the intestinal tract, which contains any selected from the group consisting of a human milk oligosaccharide and a constituent sugar of a human milk oligosaccharide.
[7] A composition for treating any selected from the group consisting of chronic kidney disease, mood disorder, and inflammatory bowel disease, which contains any selected from the group consisting of a human milk oligosaccharide and a constituent sugar of a human milk oligosaccharide.
[8] The composition according to any one of 1 to 6, wherein the control of growth is suppression of growth.
[9] A composition for use in a method for controlling growth of bacteria of the genus *Oscillibacter* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Oscillibacter* in the intestinal bacterial microbiota, a method for controlling growth of bacteria of the genus *Oscillibacter* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Oscillibacter* in the intestinal bacterial microbiota.
[10] The composition according to 9, which contains any selected from the group consisting of a fucosylated human milk oligosaccharide, a human milk oligosaccharide containing lactose as a constituent sugar, a human milk oligosaccharide containing fucose as a constituent sugar, lactose, and fucose; or the use in manufacture, method, or use according to 9, wherein the composition is such a composition.
[11] The composition according to 9 or 10, which contains any selected from the group consisting of 2'-fucosyllactose, lactose, and fucose; or the use in manufacture, method, or use according to 9 or 10, wherein the composition is such a composition.
[12] The composition according to any one of 9 to 11, which is for use as a prebiotic or synbiotic; the use in manufacture according to any one of 9 to 11, wherein the composition is such a composition; the method according to any one of 9 to 11, which comprises the step of administering the composition as a prebiotic or symbiotic; or the use according to any one of 9 to 11, wherein the composition is used as a prebiotic or symbiotic.
[13] The composition according to any one of 9 to 12, which is for treating any selected from the group consisting of chronic kidney disease, mood disorder, and inflammatory bowel disease; the use in manufacture according to any one of 9 to 12, wherein the composition is such a composition; the method according to any one of 9 to 12, which is for treating any selected from the group consisting of chronic kidney disease, mood disorder, and inflammatory bowel disease; or the use according to any one of 9 to 12, which is for such a treatment.
[14] A composition for treating a disease or condition that can be ameliorated by controlling growth of bacteria of the genus *Oscillibacter* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Oscillibacter* in the intestinal tract, a method for treating a disease or condition that can be ameliorated by controlling growth of bacteria of the genus *Oscillibacter* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Oscillibacter* in the intestinal tract.
[15] A composition for treating any selected from the group consisting of chronic kidney disease, mood disorder, and inflammatory bowel disease, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of chronic kidney disease, mood disorder, and inflammatory bowel disease, a method for treating any selected from the group consisting of chronic kidney disease, mood disorder, and inflammatory bowel disease, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of chronic kidney disease, mood disorder, and inflammatory bowel disease.
[16] The composition, use in manufacture of a composition, method, or use according to any one of 9 to 14, wherein the control of growth is suppression of growth.

The present invention provides the followings.
[1] A composition for controlling growth of bacteria of the genus *Holdemania* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid.
[3] The composition according to 1 or 2, which contains any selected from the group consisting of 2'-fucosyllactose, 3'-sialyllactose, 6'-sialyllactose, lactose, and N-acetylneuraminic acid.
[4] The composition according to any one of 1 to 3, which is for use as a prebiotic or synbiotic.
[5] The composition according to any one of 1 to 4, which is for use in treating any selected from the group consisting of mood disorder and Parkinson's disease.
[6] A composition for treating a disease or condition that can be ameliorated by controlling growth of bacteria of the genus *Holdemania* in the intestinal tract, which contains any selected from the group consisting of a human milk oligosaccharide and a constituent sugar of a human milk oligosaccharide.
[7] A composition for treating any selected from the group consisting of mood disorder and Parkinson's disease, which contains any selected from the group consisting of a human milk oligosaccharide and a constituent sugar of a human milk oligosaccharide.
[8] The composition according to any one of 1 to 6, wherein the control of growth is suppression of growth.
[9] A composition for use in a method for controlling growth of bacteria of the genus *Holdemania* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Holdemania* in the intestinal bacterial microbiota, a method for controlling growth of bacteria of the genus *Holdemania* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Holdemania* in the intestinal bacterial microbiota.
[10] The composition according to 9, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid; or the use in manufacture, method, or use according to 9, wherein the composition is such a composition.
[11] The composition according to 9 or 10, which contains any selected from the group consisting of 2'-fucosyllactose, 3'-sialyllactose, 6'-sialyllactose, lactose, and N-acetylneuraminic acid; or the use in manufacture, method, or use according to 9 or 10, wherein the composition is such a composition.
[12] The composition according to any one of 9 to 11, which is for use as a prebiotic or synbiotic; the use in manufacture according to any one of 9 to 11, wherein the composition is such a composition; the method according to any one of 9 to 11, which comprises the step of administering the composition as a prebiotic or synbiotic; or the use according to any one of 9 to 11, wherein the composition is used as a prebiotic or synbiotic.
[13] The composition according to any one of 9 to 12, which is for use in treating any selected from the group consisting of mood disorder and Parkinson's disease; the use in manufacture according to any one of 9 to 12, wherein the composition is such a composition; the method according to any one of 9 to 12, which is for treating any selected from the group consisting of mood disorder and Parkinson's disease; or the use according to any one of 9 to 12, which is for such a treatment.
[14] A composition for treating a disease or condition that can be ameliorated by controlling growth of bacteria of the genus *Holdemania* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Holdemania* in the intestinal tract, a method for treating a disease or condition that can be ameliorated by controlling growth of bacteria of the genus *Holdemania* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Holdemania* in the intestinal tract.
[15] A composition for treating any selected from the group consisting of mood disorder and Parkinson's disease, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of mood disorder and Parkinson's disease, a method for treating any selected from the group consisting of mood disorder and Parkinson's disease, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of mood disorder and Parkinson's disease.
[16] The composition, use in manufacture of a composition, method, or use according to any one of 9 to 14, wherein the control of growth is suppression of growth.

The present invention provides the followings.
[1] A composition for controlling growth of bacteria of the genus *Parasutterella* in the intestinal bacterial microbiota, which contains any selected from the group consisting of a human milk oligosaccharide and a constituent sugar of a human milk oligosaccharide.
[2] The composition according to 1, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid.
[3] The composition according to 1 or 2, which contains any selected from the group consisting of 2'-fucosyllactose, 3'-sialyllactose, 6'-sialyllactose, lactose, and N-acetylneuraminic acid.
[4] The composition according to any one of 1 to 3, which is for use as a prebiotic or synbiotic.
[5] The composition according to any one of 1 to 4, which is for use in treating any selected from the group consisting of mood disorder, irritable bowel syndrome, and liver dysfunction.
[6] A composition for treating a disease or condition that can be ameliorated by controlling growth of bacteria of the genus *Parasutterella* in the intestinal tract, which contains any selected from the group consisting of a human milk oligosaccharide and a constituent sugar of a human milk oligosaccharide.
[7] A composition for treating any selected from the group consisting of mood disorder, irritable bowel syndrome, and liver dysfunction, which contains any selected from the group consisting of a human milk oligosaccharide and a constituent sugar of a human milk oligosaccharide.
[8] The composition according to any one of 1 to 6, wherein the control of growth is suppression of growth.
[9] A composition for use in a method for controlling growth of bacteria of the genus *Parasutterella* in the intestinal bacterial microbiota, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for controlling growth of bacteria of the genus *Parasutterella* in the intestinal bacterial microbiota, a method for controlling growth of bacteria of the genus *Parasutterella* in the intestinal bacterial microbiota, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for controlling growth of bacteria of the genus *Parasutterella* in the intestinal bacterial microbiota.
[10] The composition according to 9, which contains any selected from the group consisting of a fucosylated human milk oligosaccharide, a sialylated human milk oligosaccharide, a human milk oligosaccharide containing lactose as a constituent sugar, a human milk oligosaccharide containing sialic acid as a constituent sugar, lactose, and sialic acid; or the use in manufacture, method, or use according to 9, wherein the composition is such a composition.
[11] The composition according to 9 or 10, which contains any selected from the group consisting of 2'-fucosyllactose, 3'-sialyllactose, 6'-sialyllactose, lactose, and N-acetylneuraminic acid; or the use in manufacture, method, or use according to 9 or 10, wherein the composition is such a composition.
[12] The composition according to any one of 9 to 11, which is for use as a prebiotic or synbiotic; the use in manufacture according to any one of 9 to 11, wherein the composition is such a composition; the method according to any one of 9 to 11, which comprises the step of administering the composition as a prebiotic or synbiotic; or the use according to any one of 9 to 11, wherein the composition is used as a prebiotic or synbiotic.
[13] The composition according to any one of 9 to 12, which is for use in treating any selected from the group consisting of mood disorder, irritable bowel syndrome, and hepatic dysfunction; the use in manufacture according to any one of 9 to 12, wherein the composition is such a composition; the method according to any one of 9 to 12, which is for treating any selected from the group consisting of mood disorder, irritable bowel syndrome, and hepatic dysfunction; or the use according to any one of 9 to 12, which is for such a treatment.
[14] A composition for treating a disease or condition that can be ameliorated by controlling growth of bacteria of the genus *Parasutterella* in the intestinal tract, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating a disease or condition that can be ameliorated by controlling growth of bacteria of the genus *Parasutterella* in the intestinal tract, a method for treating a disease or condition that can be ameliorated by controlling growth of bacteria of the genus *Parasutterella* in the intestinal tract, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating a disease or condition that can be ameliorated by controlling growth of bacteria of the genus *Parasutterella* in the intestinal tract.
[15] A composition for treating any selected from the group consisting of mood disorder, irritable bowel syndrome, and hepatic dysfunction, which contains any selected from the group consisting of a human milk oligosaccharide and a constituent sugar of a human milk oligosaccharide, use of any selected from the group consisting of a human milk oligosaccharide and a constituent sugar of a human milk oligosaccharide in manufacture of a composition for treating any selected from the group consisting of mood disorder, irritable bowel syndrome, and hepatic dysfunction, a method for treating any selected from the group consisting of mood disorder, irritable bowel syndrome, and hepatic dysfunction, which comprises the step of administering a composition containing any selected from the group consisting of a human milk oligosaccharide and a constituent sugar of a human milk oligosaccharide to a subject, or use of a composition containing any selected from the group consisting of a human milk oligosaccharide and a constituent sugar of a human milk oligosaccharide for treating any selected from the group consisting of mood disorder, irritable bowel syndrome, and hepatic dysfunction.
[16] The composition, use in manufacture of a composition, method, or use according to any one of 9 to 14, wherein the control of growth is suppression of growth.

### Effects of the Invention

According to the present invention, intestinal bacterial microbiota can be controlled. In particular, growth or occupancy rate in the bacterial microbiota of bacteria of the genus *Eggerthella,* bacteria of the genus *Oscillibacter,* bacteria of the genus *Holdemania,* and bacteria of the genus *Parasutterella* can be controlled.

Through controlling growth or increase in occupancy rate in the bacterial microbiota of bacteria of the genus *Eggerthella* with specific active ingredients, any selected from the group consisting of inflammatory bowel disease, multiple sclerosis, rheumatoid arthritis, mood disorder, and asthma can be treated, and such active ingredients can be administered to a patient with colitis aggravated by activation of helper T17 cells, or administered to a patient with heart failure or arrhythmia who is being administered digoxin. According to the present invention, it can also be expected to treat various diseases or conditions that can be ameliorated by controlling increase of bacteria of the genus *Eggerthella* in the intestinal bacterial microbiota.

Through controlling growth or increase in occupancy rate in the bacterial microbiota of bacteria of the genus *Oscillibacter* with specific active ingredients, any selected from the group consisting of chronic kidney disease, mood disorder, and inflammatory bowel disease can be treated. According to the present invention, it can also be expected to treat various diseases or conditions that can be ameliorated by controlling increase of bacteria of the genus *Oscillibacter* in the intestinal bacterial microbiota.

Through controlling growth or increase in occupancy rate in the bacterial microbiota of bacteria of the genus *Holdemania* with specific active ingredients, any selected from the group consisting of mood disorder and Parkinson's disease can be treated. According to the present invention, it can also be expected to treat various diseases or conditions that can be ameliorated by controlling increase of bacteria of the genus *Holdemania* in the intestinal bacterial microbiota.

Through controlling growth or increase in occupancy rate in the bacterial microbiota of bacteria of the genus *Parasutterella* with specific active ingredients, any selected from the group consisting of mood disorder, irritable bowel syndrome, and hepatic dysfunction can be treated. According to the present invention, it can also be expected to treat various diseases or conditions that can be ameliorated by controlling increase of bacteria of the genus *Parasutterella* in the intestinal bacterial microbiota.

By adding the active ingredients of the present invention, functional food products in various forms can be provided.

### Brief Description of the Drawings

[Fig. 1] Changes in occupancy rate of bacteria of the genus *Eggerthella* induced by various carbohydrates in a human fecal fermentation system, evaluated by the Wilcoxon signed rank test.
[Fig. 2] Changes in occupancy rate of bacteria of the genus *Oscillibacter* induced by various carbohydrates in a human fecal fermentation system, evaluated by the Wilcoxon signed rank test or paired t-test.
[Fig. 3] Changes in occupancy rate of bacteria of the genus *Oscillibacter* induced by fucose in a human fecal fermentation system, evaluated by the Wilcoxon signed rank test.
[Fig. 4] Changes in occupancy rate of bacteria of the genus *Holdemania* induced by various carbohydrates in a human fecal fermentation system, evaluated by the paired t-test or Wilcoxon signed rank test.
[Fig. 5] Change in occupancy rate of bacteria of the genus *Parasutterella* induced by various carbohydrates in a human fecal fermentation system, evaluated by the Wilcoxon signed rank test.
[Fig. 6] Change in occupancy rate of bacteria of the genus *Parasutterella* induced by 6'-SL in a human fecal fermentation system, evaluated by the paired t-test.

### Modes for Carrying out the Invention

Hereafter, the present invention will be explained in detail.

The present invention relates to a composition for controlling growth of bacteria of the genus *Eggerthella,* bacteria of the genus *Oscillibacter,* bacteria of the genus *Holdemania,* and bacteria of the genus *Parasutterella* in the intestinal bacterial microbiota, which contains any selected from the group consisting of a human milk oligosaccharide (also referred to as HMO) and a constituent sugar of a human milk oligosaccharide as an active ingredient.

### [Active Ingredient]

The composition of the present invention contains any selected from the group consisting of a human milk oligosaccharide (also referred to as HMO) and a constituent sugar of a human milk oligosaccharide as an active ingredient. Human milk oligosaccharides are oligosaccharides contained in human milk and are the third most abundant solid component after lactose and lipids. With few exceptions, human milk oligosaccharides have a chemical structure comprising a lactose unit at the reducing end, to which fucose, sialic acid, galactose and so forth are added.

For the present invention, the abbreviation and structure of milk oligosaccharides are according to the descriptions of Nutr Rev. 2017 Nov 1;75(11):920-933. doi: 10.1093/nutrit/nux044; and Nutrients 2021, 13(8), 2737; https://doi.org/10.3390/nu13082737. In case of discrepancies between these references, the latter should be followed.

The human milk oligosaccharide and constituent sugar of a human milk oligosaccharide used in the composition are not particularly limited so long as they can provide the desired effect. The human milk oligosaccharide and constituent sugar of a human milk oligosaccharide used in the composition may consist of one type or a combination of two or more types thereof.

Examples of the human milk oligosaccharide contained in the composition include the following neutral and acidic oligosaccharides
Neutral oligosaccharides: 2'-fucosyllactose (2'-FL), 3-fucosyllactose (3-FL), difucosyllactose (DFL), lacto-difucotetraose (LDFT), lacto-N-tetraose (LNT), lacto-N-neotetraose (LNnT), lacto-N-fucopentaose I (LNFP I), lacto-N-fucopentaose II (LNFP II), lacto-N-fucopentaose III (LNFP III), lacto-N-fucopentaose V (LNFP V), lacto-N-fucopentaose VI (LNFP VI), lacto-N-difucohexaose I (LNDFH I), lacto-N-difucohexaose II (LNDFH II), lacto-N-hexaose (LNH), para-lacto-N-hexaose (pLNH), lacto-N-neohexaose (LNnH), para-lacto-N-neohexaose (pLNnH), fucosyllacto-N-hexaose I (F-LNH I), fucosyl-para-lacto-N-hexaose I (F-para-LNH I), fucosyllacto-N-hexaose II (F-LNH II), fucosyllacto-N-hexaose III (F-LNH III), difucosyllacto-N-hexaose I (DF-LNH I), difucosyllacto-N-hexaose II (DF-LNH II), difucosyllacto-N-hexaose III (DF-LNH III), difucosyl-para-lacto-N-neohexaose (DF -pLNnH), difucosyl-para-lacto-N-hexaose (DF-para-LNH), difucosyl-para-lacto-N-neohexaose (DF-para-LNnH), and trifucosyllacto-N-hexaose (TF-LNH).
Acidic oligosaccharides: 3'-sialyllactose (3'-SL), 6'-sialyllactose (6'-SL), 6'-sialyl-N-acetyllactosamine (6'-SLN), LS-tetrasaccharide a (LST a, sialyllacto-N-tetraose a), LS-tetrasaccharide b (LST b, sialyllacto-N-tetraose b), LS-tetrasaccharide c (LST c, sialyllacto-N-tetraose c), disialyllacto-N-tetraose (DS-LNT), fucosyl-sialyllacto-N-tetraose a (F-LST a), fucosyl-sialyllacto-N-tetraose b (F-LST b), fucosyl-sialyllacto-N-hexaose (FS-LNH), fucosyl-sialyllacto-N-neohexaose (FS-LNnH I), fucosyl-disialyllacto-N-hexaose (FDS-LNH), and disialyllacto-N-hexaose (DS-LNH).
Preferred examples of the human milk oligosaccharide contained in the composition include the following neutral and acidic oligosaccharides.
   Neutral oligosaccharides: 2'-fucosyllactose (2'-FL), 3-fucosyllactose (3-FL), difucosyllactose (DFL), lacto-difucotetraose (LDFT), lacto-N-tetraose (LNT), lacto-N-neotetraose (LNnT), lacto-N-fucopentaose I (LNFP I), lacto-N-fucopentaose II (LNFP II), lacto-N-fucopentaose III (LNFP III), lacto-N-fucopentaose V (LNFP V), lacto-N-fucopentaose VI (LNFP VI), lacto-N-difucohexaose I (LNDFH I), lacto-N-difucohexaose II (LNDFH II), lacto-N-hexaose (LNH), lacto-N-neohexaose (LNnH), para-lacto-N-neohexaose (pLNnH), fucosyllacto-N-hexaose I (F-LNH I), fucosyl-para-lacto-N-hexaose I (F-para-LNH I), fucosyllacto-N-hexaose II (F-LNH II), fucosyllacto-N-hexaose III (F-LNH III), difucosyllacto-N-hexaose I (DF-LNH I), difucosyllacto-N-hexaose II (DF-LNH II), difucosyllacto-N-hexaose III (DF-LNH III), difucosyl-para-lacto-N-neohexaose (DF-pLNnH), and trifucosyllacto-N-hexaose (TF-LNH).
Acidic oligosaccharides: 3'-sialyllactose (3'-SL), 6'-sialyllactose (6'-SL), LS-tetrasaccharide a (LST a, sialyllacto-N-tetraose a), LS-tetrasaccharide b (LST b, sialyllacto-N-tetraose b), LS-tetrasaccharide c (LST c, sialyllacto-N-tetraose c), disialyllacto-N-tetraose (DS-LNT), fucosyl-sialyllacto-N-neohexaose (FS-LNnH I), and disialyllacto-N-hexaose (DS-LNH).

More preferred examples of the human milk oligosaccharide contained in the composition include the following neutral and acidic oligosaccharides.
Neutral oligosaccharides: 2'-fucosyllactose (2'-FL), difucosyllactose (DFL, lacto-difucotetraose (LDFT)), lacto-N-tetraose (LNT), and lacto-N-neotetraose (LNnT). Acidic oligosaccharides: 3'-sialyllactose (3'-SL), and 6'-sialyllactose (6'-SL)

For the present invention, the term constituent sugar of a human milk oligosaccharide refers to a sugar constituting a human milk oligosaccharide (sugar resulting from degradation of human milk oligosaccharide), excluding glucose and galactose. Examples of the constituent sugar of a human milk oligosaccharide contained in the composition include lactose, fucose, sialic acid, and N-acetylglucosamine. Sialic acid is a general term for modifications of neuraminic acid. An example of sialic acid is N-acetylneuraminic acid (Neu5Ac), which is an acetyl-modified neuraminic acid.

According to the studies of the inventors of the present invention, there were confirmed superior growth-suppressing effects of lactose, fucose, 2'-FL, 3'-SL, and 6'-SL for bacteria of the genus *Eggerthella.* By the way, human milk oligosaccharides can generate lactose in the human digestive tract after ingestion. In addition, human milk oligosaccharides containing fucose as a constituent sugar can generate fucose in the human digestive tract after ingestion (see Non-patent documents 13 and 14 mentioned later). Furthermore, since the desired effect was confirmed for all of 2'-FL, 3'-SL, and 6'-SL, the desired effect would be sufficiently obtained with fucosylated human milk oligosaccharides and sialylated human milk oligosaccharides.

From this point of view, preferred examples of the human milk oligosaccharide and constituent sugar of a human milk oligosaccharide used in the composition are any selected from the group consisting of fucosylated human milk oligosaccharides, sialylated human milk oligosaccharides, human milk oligosaccharides containing lactose as a constituent sugar, human milk oligosaccharides containing fucose as a constituent sugar, lactose, and fucose. Human milk oligosaccharides containing fucose as a constituent sugar include fucosylated human milk oligosaccharides (e.g., 2'-FL), and fucose-containing human milk oligosaccharides (e.g., DFL (LDFT)).

Specific examples of the human milk oligosaccharide and constituent sugar of a human milk oligosaccharide used in the composition are any selected from the group consisting of 2'-FL, 3'-SL, 6'-SL, lactose, and fucose.

According to the studies of the inventors of the present invention, there were confirmed superior growth-suppressing effects of lactose, fucose, and 2'-FL for bacteria of the genus *Oscillibacter.* By the way, human milk oligosaccharides can generate lactose in the human digestive tract after ingestion. In addition, human milk oligosaccharides containing fucose as a constituent sugar can generate Neu5Ac and fucose in the human digestive tract after ingestion (see Non-patent documents 13 and 14 mentioned later). Since the desired effect was confirmed for 2'-FL, the desired effect would be sufficiently obtained with fucosylated human milk oligosaccharides.

From this point of view, preferred examples of the human milk oligosaccharide and constituent sugar of a human milk oligosaccharide used in the composition are any selected from the group consisting of fucosylated human milk oligosaccharides, sialylated human milk oligosaccharides, human milk oligosaccharides containing lactose as a constituent sugar, human milk oligosaccharides containing fucose as a constituent sugar, lactose, and fucose. Human milk oligosaccharides containing fucose as a constituent sugar include fucosylated human milk oligosaccharides (e.g., 2'-FL) and fucose-containing human milk oligosaccharides (e.g., DFL (LDFT)).

Specific examples of the human milk oligosaccharide and constituent sugar of a human milk oligosaccharide used in the composition are any selected from the group consisting of 2'-FL, lactose, and fucose.

According to the studies of the inventors of the present invention, there were confirmed superior growth-suppressing effects of lactose, Neu5Ac, 2'-FL, 3'-SL, and 6'-SL for bacteria of the genus *Holdemania.* By the way, human milk oligosaccharides can generate lactose in the human digestive tract after ingestion. In addition, human milk oligosaccharides containing Neu5Ac as a constituent sugar can generate Neu5Ac in the human digestive tract after ingestion (see Non-patent documents 13, 15 and 16 mentioned later). Furthermore, since the desired effect was confirmed for all of 2'-FL, 3'-SL, and 6'-SL, the desired effect would be sufficiently obtained with fucosylated human milk oligosaccharides and sialylated human milk oligosaccharides.

From this point of view, preferred examples of the human milk oligosaccharide and constituent sugar of a human milk oligosaccharide used in the composition are any selected from the group consisting of fucosylated human milk oligosaccharides, sialylated human milk oligosaccharides, human milk oligosaccharides containing lactose as a constituent sugar, human milk oligosaccharides containing sialic acid as a constituent sugar, lactose, and sialic acid. Human milk oligosaccharides containing sialic acid as a constituent sugar include sialylated human milk oligosaccharides (e.g., 3'-SL and 6'-SL) and sialic acid-containing human milk oligosaccharides (e.g., LST a and DS-LNT).

Specific examples of the human milk oligosaccharide and constituent sugar of a human milk oligosaccharide used in the composition are any selected from the group consisting of 2'-FL, 3'-SL, 6'-SL, lactose, and Neu5Ac.

According to the studies of the inventors of the present invention, there were confirmed superior growth-suppressing effects of lactose, Neu5Ac, 2'-FL, 3'-SL, and 6'-SL for bacteria of the genus *Parasutterella.* By the way, human milk oligosaccharides can generate lactose in the human digestive tract after ingestion. In addition, human milk oligosaccharides containing Neu5Ac as a constituent sugar can generate Neu5Ac in the human digestive tract after ingestion (see Non-patent documents 13, 15 and 16 mentioned later). Furthermore, since the desired effect was confirmed for all of 2'-FL, 3'-SL, and 6'-SL, the desired effect would be sufficiently obtained with fucosylated human milk oligosaccharides and sialylated human milk oligosaccharides.

From this point of view, preferred examples of the human milk oligosaccharide and constituent sugar of a human milk oligosaccharide used in the composition are any selected from the group consisting of fucosylated human milk oligosaccharides, sialylated human milk oligosaccharides, human milk oligosaccharides containing lactose as a constituent sugar, human milk oligosaccharides containing sialic acid as a constituent sugar, lactose, and sialic acid. Human milk oligosaccharides containing sialic acid as a constituent sugar include sialylated human milk oligosaccharides (e.g., 3'-SL and 6'-SL) and sialic acid-containing human milk oligosaccharides (e.g., LST a and DS-LNT).

Specific examples of the human milk oligosaccharide and constituent sugar of a human milk oligosaccharide used in the composition are any selected from the group consisting of 2'-FL, 3'-SL, 6'-SL, lactose, and Neu5Ac.

### [Use]

The composition of the present invention can be used to control growth of bacteria known to be associated with mental and behavioral disorders in the intestinal bacterial microbiota. The composition of the present invention can be used to control growth of any bacteria selected from the group consisting of bacteria of the genus *Eggerthella,* bacteria of the genus *Oscillibacter,* bacteria of the genus *Holdemania,* and bacteria of the genus *Parasutterella* in intestinal bacterial microbiota. The control of growth includes promotion of growth and suppression of growth. Preferred compositions are for suppression of growth.

Examples of bacteria of the intestinal bacterial microbiota include the followings:
bacteria of the genus *Faecalibacterium,* e.g., *Faecalibacterium prausnitzii*;
bacteria of the genus *Akkermansia,* e.g., *Akkermansia muciniphila*;
bacteria of the genus *Blautia,* e.g., *Blautia coccoides, Blautia intestinalis,* and *Blautia faecicola*;
bacteria of the genus *Subdoligranulum,* e.g., *Subdoligranulum variabile*;
bacteria of the genus *Bilophila* e.g., *Bilophila wadsworthia*;
bacteria of the genus *Collinsella,* e.g., *Collinsella aerofaciens* and *Collinsella intestinalis*;
bacteria of the genus *Holdemania,* e.g., *Holdemania filiformis*;
bacteria of the genus *Parasutterella,* e.g., *Parasutterella excrementihominis* and *Parasutterella secunda*;
bacteria of the genus *Oscillibacter,* e.g., *Oscillibacter valericigenes*;
bacteria of the genus *Eggerthella,* e.g., *Eggerthella lenta*;
bacteria of the genus *Sutterella,* e.g., *Sutterella parvirubra* and *Sutterella wadsworthensis*; and
bacteria of the genus *Bifidobacterium* (bifidobacteria), e.g., *B. adolescentis, B. bifidum, B. breve, B. catenulatum, B. infantis, B. lactis, B. longum,* and *B. pseudocatenulatum.*

For the present invention, the term bacteria of the genus *Eggerthella* refers to bacteria identified as belonging to the genus *Eggerthella* by molecular phylogenetic analysis based on the 16S rRNA gene. For the present invention, the term bacteria of the genus *Oscillibacter* refers to bacteria identified as belonging to the genus *Oscillibacter* by molecular phylogenetic analysis based on the 16S rRNA gene. For the present invention, the term bacteria of the genus *Holdemania* refers to bacteria identified as belonging to the genus *Holdemania* by molecular phylogenetic analysis based on the 16S rRNA gene. For the present invention, the term bacteria of the genus *Parasutterella* refers to bacteria identified as belonging to the genus *Parasutterella* by molecular phylogenetic analysis based on the 16S rRNA gene. Criteria for determining genus by molecular phylogenetic analysis based on the 16S rRNA gene are well known by those skilled in the art (Stackebrandt E, Ebers J. Taxonomic parameters revisited: tarnished gold standards. Microbiol Today. 2006;33:152-155).

For the present invention, controlling growth of specific bacteria in bacterial microbiota means controlling the proportion (occupancy rate) of the specific bacteria in the bacterial microbiota.

Whether or not a certain ingredient controls growth of a particular type of bacteria in the intestinal bacterial microbiota can be evaluated as follows. A fecal material provided by a healthy person is added to an appropriate medium, cultured for a certain period of time as required, then an ingredient to be evaluated is added, cultured under appropriate conditions (e.g., at 37°C under anaerobic conditions, similar to those in the intestinal tract, for 48 hours), and the occupancy rate of the particular type of bacteria in the bacterial microbiota in the culture is measured. The result of the measurement can then be compared with a result of a culture obtained under conditions that differ only in that a control (e.g., sterile water) is added in place of the ingredient to be evaluated but that are otherwise identical, and it can be determined that, if the occupancy rate is higher than the control, growth is promoted, and if lower, growth is suppressed.

Occupancy rate of a particular type of bacteria in bacterial microbiota can be determined by known methods. One preferred method is to perform 16S metagenomic analysis (sequence analysis of 16S rRNA gene amplicons) on DNAs extracted from culture. When the 16S metagenomic analysis is performed, DNA extraction can be performed by using commercially available kits. The genomic region to be analyzed is not particularly limited so long as the bacteria can be determined, but the V3-V4 region of the 16S rRNA gene can be used. Primers, amplification conditions, method for purification of amplicons, and so forth well known to those skilled in the art can be used to analyze bacteria. Sequence decoding is preferably performed on a next-generation sequencer of higher performance. A next-generation microbiome bioinformatics platform such as QIIME 2^{™} can be used to analyze the obtained data. For the 16S metagenomic analysis, those skilled in the art can refer to such information as Sanschagrin S, Yergeau E. Next-generation sequencing of 16S ribosomal RNA gene amplicons. J Vis Exp. 2014;(90):51709. Published 2014 Aug 29. doi: 10.3791/51709.

It has been reported that there is a relationship between intestinal bacteria and mood disorder such as depression. For example, it has been reported that when feces from depressed patients and normal subjects were administered to germ-free mice, depression-like behaviors increased in mice treated with feces from depressed patients compared with those treated with feces from normal subjects (Non-patent document 1 mentioned above). In addition, a systematic review comparing the intestinal bacteria of healthy and depressed individuals reported that intestinal bacteria of 24 genera in total, including bacteria of the genera *Eggerthella, Oscillibacter, Holdemania,* and *Parasutterella,* were more abundant in depressed individuals (Non-patent document 2 mentioned above). Therefore, the composition of the present invention that can control growth of specific intestinal bacteria can be used for treating any selected from the group consisting of depression and mood disorder.

Bacteria of the genus *Eggerthella* as one of the objects of the present invention are one class of commensal bacteria in the human intestine, which have recently been reported to be associated with various diseases. For example, it has been reported that bacteria of the genus *Eggerthella* have a high occupancy rate in patients with inflammatory bowel disease, multiple sclerosis, and rheumatoid arthritis (Forbes JD, Chen CY, Knox NC, et al. A comparative study of the intestinal bacterial microbiota in immune-mediated inflammatory diseases-does a common dysbiosis exist?. Microbiome. 2018;6(1):221. Published 2018 Dec 13. doi:10.1186/s40168-018-0603-4). A high occupancy rate of bacteria of the genus *Eggerthella* has also been reported in asthma-affected individuals (Wang Q, Li F, Liang B, et al. A metagenome-wide association study of intestinal bacterial microbiota in asthma in UK adults. BMC Microbiol. 2018;18(1):114. Published 2018 Sep 12. doi:10.1186/s12866-018-1257-x). Furthermore, it has also been reported that bacteria of the genus *Eggerthella* aggravate colitis by activating helper T17 cells (Alexander M, Ang QY, Nayak RR, et al. Human gut bacterial metabolism drives Th17 activation and colitis [published online ahead of print, 2021 Nov 18]. Cell Host Microbe. 2021;S1931-3128(21)00507-2. doi:10.1016/j.chom.2021.11.001), and inactivate the inotropic drug digoxin for treating heart failure and arrhythmia (Haiser HJ, Gootenberg DB, Chatman K, Sirasani G, Balskus EP, Turnbaugh PJ. Predicting and manipulating cardiac drug inactivation by the human gut bacterium Eggerthella lenta. Science. 2013;341(6143):295-298. doi:10.1126/science.1235872). Therefore, the composition can be used for treating any selected from the group consisting of inflammatory bowel disease, multiple sclerosis, rheumatoid arthritis, mood disorder, and asthma, for administration to a patient with colitis exacerbated by activation of helper T17 cells, or for administration to a patient with heart failure or arrhythmia who is being administered digoxin.

Bacteria of the genus *Oscillibacter* as one of the objects of the present invention are a class of commensal bacteria in the human intestine, which have recently been reported to be associated with various diseases. For example, the occupancy rate of bacteria of the genus *Oscillibacter* increases in patients with chronic kidney disease, and a positive correlation thereof with urinary toxicants has been demonstrated (Kim JE, Kim HE, Park JI, et al. The Association between Gut Microbiota and Uremia of Microorganisms. 2020;8(6):907. Published 2020 Jun 16. doi:10.3390/microorganisms8060907). It has also been reported that the occupancy rate of bacteria of the genus *Oscillibacter* is also high in mouse models of inflammatory bowel disease (Wang C, Li W, Wang H, et al. Saccharomyces boulardii alleviates ulcerative colitis carcinogenesis in mice by reducing TNF-α and IL-6 levels and functions and by rebalancing intestinal microbiota. BMC Microbiol. 2019;19(1):246. Published 2019 Nov 6. doi:10.1186/s12866-019-1610-8). Therefore, the composition can be used for treating any selected from the group consisting of chronic kidney disease, mood disorder, and inflammatory bowel disease.

Bacteria of the genus *Holdemania* as one of the objects of the present invention have also been reported to be abundant in the intestines of Parkinson's disease patients (Alteration of the fecal microbiota in Chinese patients with Parkinson's disease. Brain Behav Immun. 2018;70:194-202. doi:10.1016/j.bbi.2018.02.016). Therefore, the composition can be used for treating any selected from the group consisting of mood disorder and Parkinson's disease.

Bacteria of the genus *Parasutterella* as one of the objects of the present invention are found in the intestines of humans as well as a variety of animals, such as mice, dogs, and chickens. Bacteria of the genus *Parasutterella* have been reported to show high occupancy rates in patients suffering from irritable bowel syndrome (IBS) (Chen YJ, Wu H, Wu SD, et al. Parasutterella, in association with irritable bowel syndrome and intestinal chronic inflammation. J Gastroenterol Hepatol. 2018;33(11):1844-1852. doi:10.1111/jgh.14281), major depression or depression (Non-patent documents 9 and 10 mentioned above), and associate with liver dysfunction due to a high-fat diet (Blasco-Baque V, Coupe B, Fabre A, et al. Associations between hepatic miRNA expression, liver triacylglycerols and gut microbiota during metabolic adaptation to high-fat diet in mice. Diabetologia. 2017;60(4):690-700. doi:10.1007/s00125-017-4209-3). Therefore, the composition can be used for treating any selected from the group consisting of mood disorder, irritable bowel syndrome, and liver dysfunction.

Mood disorder is classified as "mental and behavioral disorder" in the 11th revision of the International Classification of Diseases (ICD-11). Mood disorder (sometimes referred to as affective disorder, ICD-11, F30-F39) is a general term for conditions that cause significant disturbances in daily life due to fluctuations in mood (emotion), and includes depression, bipolar disorder (manic-depressive illness), and mood dysregulation. Depression is a condition in which mental symptoms such as feeling depressed all day long and not being able to enjoy anything, as well as physical symptoms such as difficulty sleeping, loss of appetite, and tiredness appear and cause significant disturbances in daily life. Depressed state (sometimes called depressive state) is a state in which a depressive mood (a negative emotional state characterized by depression, sadness, emptiness, hopelessness, or despondency) persists, for example, a depressed mood lasts almost all day, almost every day, for two weeks or longer.

Inflammatory bowel disease includes ulcerative colitis and Crohn's disease. Multiple sclerosis is a disease in which immune cells cause inflammation of the central nervous system (brain and spinal cord) and optic nerves, resulting in nerve tissue damage, and is one of autoimmune diseases. Rheumatoid arthritis is a disease that produces synovial inflammation in joints and is one of autoimmune diseases. Chronic kidney disease is diagnosed when kidney function is reduced to 60% or less of healthy persons (eGFR lower than 60 ml/min/1.73 m²) or when kidney abnormalities such as proteinuria persist for three months or longer. There are many causes of chronic kidney disease, which include, for example, diabetes, hypertension, obesity, metabolic syndrome, diseases of the kidneys themselves such as chronic nephritis syndrome and nephrotic syndrome, collagen disease, infectious diseases, hereditary abnormalities, medication (e.g., administration of antipyretic analgesics, Chinese medicine, anticancer drugs, etc.), aging, and so forth. Inflammatory bowel disease includes ulcerative colitis and Crohn's disease. Parkinson's disease includes those that develop at age 50 or older and juvenile Parkinson's disease.

The composition can also be used for treating a disease or condition that can be ameliorated by controlling growth of any bacteria selected from the group consisting of bacteria of the genus *Eggerthella,* bacteria of the genus *Oscillibacter,* bacteria of the genus *Holdemania,* and bacteria of the genus *Parasutterella* in the intestinal tract. Such a disease or condition can be mood disorder as well as other various mental and behavioral disorders.

The composition can also be used for treating a disease or condition that can be ameliorated by controlling growth of bacteria of the genus *Eggerthella* in the intestinal tract. Such a disease or condition may be inflammatory bowel disease, multiple sclerosis, rheumatoid arthritis, mood disorder, asthma, colitis, heart failure and arrhythmia, as well as many others.

The composition can also be used for treating a disease or condition that can be ameliorated by controlling growth of bacteria of the genus *Oscillibacter* in the intestinal tract. Such a disease or condition may be chronic kidney disease, mood disorder, and inflammatory bowel disease, as well as many others.

The composition can also be used for treating a disease or condition that can be ameliorated by controlling growth of bacteria of the genus *Holdemania* in the intestinal tract. Such a disease or condition may be mood disorder and Parkinson's disease, as well as many others.

The composition can also be used for treating a disease or condition that can be ameliorated by controlling growth of bacteria of the genus *Parasutterella* in the intestinal tract. Such a disease or condition may be mood disorder, irritable bowel syndrome, and liver dysfunction, as well as many others.

For the present invention, treating (treatment of) a disease or condition includes reducing the risk of developing the disease or condition, delaying or preventing the development of the disease or condition, and stopping or delaying the progression of the disease or condition. The therapy includes curative therapy (therapy to remove the cause) and symptomatic therapy (therapy to ameliorate symptoms, e.g., in the case of depression, therapies to ameliorate physical discomforts such as loss of appetite, insomnia, fatigue, headache, stiff shoulders, and palpitations). Actions for amelioration or treatment include medical actions performed by physicians and nurses, midwives, and others under the direction of physicians, and non-therapeutic actions performed by non-physicians, such as pharmacists, nutritionists (including registered dietitian nutritionists and sports nutritionists), public health nurses, midwives, nurses, clinical laboratory technicians, sports instructors, drug manufacturers, drug sellers, food manufacturers, food sellers, and others. Furthermore, prevention or reduction of the risk of developing disease or condition includes recommendations for intake of specific foods and nutritional guidance (including guidance on nutrition necessary for medical treatment of injured or sick persons and guidance on nutrition for the maintenance and promotion of good health).

### [Composition]

### (Food compositions, etc.)

The composition of the present invention can be in the form of a food composition or pharmaceutical composition. Food and pharmaceutical are not limited to those for humans, and may be those for animals other than human, unless especially indicated. The food may be a common food, functional food, or nutritional composition, or a therapeutic diet (diet for the purpose of therapy, for which a medical practitioner writes a dietary prescription, and which is cooked by a dietitian or the like according to the prescription), dietetic food, ingredient-modified food, care food, or therapy-supporting food, unless especially indicated. The food is not limited to a solid food, but it may be a food in the form of liquid, for example, drink, drinkable preparation, liquid food, or soup, unless especially indicated. Functional food refers to a food that can give a predetermined functionality to a living body, and includes health foods at large, such as foods for specified health uses (abbreviated as "Tokuho" in Japanese, including conditional foods for specified health use), foods with function claims, foods with health claims including foods with nutrient function claims, foods for special dietary uses, supplements (for example, those of various kinds of dosage forms such as tablet, coated tablet, sugar-coated tablet, capsule and solution), and cosmetic food (for example, diet foods). In the present invention, the "functional foods" include health foods to which the health claim based on the food standards of CODEX (JOINT FAO/WHO FOOD STANDARDS PROGRAMME CODEX ALIMENTARIUS COMMISSION) is applied.

### (Subject)

The composition of the present invention is suitable to be administered to subjects for whom it is desirable or necessary to control growth of bacteria of the genus *Eggerthella* in the intestinal tract; persons with any selected from the group consisting of inflammatory bowel disease, multiple sclerosis, rheumatoid arthritis, mood disorder, and asthma, patients of colitis aggravated by activation of helper T17 cells, or patients of heart failure or arrhythmia who are being administered digoxin; and subjects with a disease or condition that can be ameliorated by controlling growth of bacteria of the genus *Eggerthella* in the intestinal tract. Subjects for whom it is desirable or necessary to control growth of bacteria of the genus *Eggerthella* in the intestinal tract include those with high (or excessive) numbers of bacteria of the genus *Eggerthella* in the intestinal tract.

The composition of the present invention is suitable to be administered to subjects for whom it is desirable or necessary to control growth of bacteria of the genus *Oscillibacter* in the intestinal tract; persons with any selected from the group consisting of chronic kidney disease, mood disorder, and inflammatory bowel disease; and subjects with a disease or condition that can be ameliorated by controlling growth of bacteria of the genus *Oscillibacter* in the intestinal tract. Subjects for whom it is desirable or necessary to control growth of bacteria of the genus *Oscillibacter* in the intestinal tract include those with high (or excessive) numbers of bacteria of the genus *Oscillibacter* in the intestinal tract.

The composition of the present invention is suitable to be administered to subjects for whom it is desirable or necessary to control growth of bacteria of the genus *Holdemania* in the intestinal tract; subjects with any selected from the group consisting of mood disorder and Parkinson's disease; and subjects with a disease or condition that can be ameliorated by controlling growth of bacteria of the genus *Holdemania* in the intestinal tract. Subjects for whom it is desirable or necessary to control growth of bacteria of the genus *Holdemania* in the intestinal tract include those with high (or excessive) numbers of bacteria of the genus *Holdemania* in the intestinal tract.

The composition of the present invention is suitable to be administered to subjects for whom it is desirable or necessary to control growth of bacteria of the genus *Parasutterella* in the intestinal tract; subjects with any selected from the group consisting of mood disorder, irritable bowel syndrome, and hepatic dysfunction; and subjects with a disease or condition that can be ameliorated by controlling growth of bacteria of the genus *Parasutterella* in the intestinal tract. Subjects for whom it is desirable or necessary to control growth of bacteria of the genus *Parasutterella* in the intestinal tract include those with high (or excessive) numbers of bacteria of the genus *Parasutterella* in the intestinal tract.

For the present invention, the term administration is used in the sense of administering a pharmaceutical product to a subject as well as having a subject ingest a food or other substance other than a pharmaceutical product.

The age of the subject is not particularly limited, and the subject may be, for example, a neonate (within 28 days of birth); an infant (younger than 1 year of age); a young child (1 to 6 years of age); a child (7 years of age or older and younger than 15 years of age); an adult (15 years of age or older); or a person of 65 years of age or older.

### (Administration route etc.)

The composition of the present invention may be administered orally, parenterally, e.g., via a tube (gastrostomy or enterostomy), or intranasally, but oral administration is preferred.

The composition can be administered to the subject repeatedly or continuously over a long period of time. The period of time is not particularly limited, but in order for the effect to be fully recognized, the composition should be administered continuously over a relatively long period of time, e.g., over 3 days or longer, 1 week or longer, 2 weeks or longer, 1 month or longer, 3 months or longer, 6 months or longer, or 1 year or longer.

The composition may be administered routinely, in advance, such as when the risk is high, or when the need arises. The composition may be administered as a meal, before, after, or between meals, or when the disease or condition that is to be ameliorated by the composition occurs.

### (Dose and content)

The dose of the composition of the present invention may be such an amount that the desired effect is exerted. The dose can be appropriately set in consideration of various factors such as the age, weight, and symptoms of the subject.

The daily dose of the composition can be at least 0.1 mg, preferably at least 0.3 mg, more preferably at least 0.6 mg, even more preferably at least 1 mg, in terms of the amount of the active ingredient. The maximum amount of the active ingredient per day may be 10 g or less, 5 g or less, 1 g or less, 100 mg or less, 60 mg or less, 30 mg or less, or 15 mg or less, no matter how the minimum amount is defined. When two or more types of active ingredients are contained in the composition, the amount of active ingredient means the total amount of the active ingredients contained.

Administration may be once a day or multiple times a day, e.g., 2 to 10 times. The dose of the active ingredient per one time may be, for example, 0.01 mg or more, preferably 0.03 mg or more, more preferably 0.06 mg or more, further preferably 0.1 mg or more. The maximum amount of the active ingredient per one time may be 3 g or less, 1 g or less, 100 mg or less, 33 mg or less, 20 mg or less, 10 mg or less, or 5 mg or less, no matter how is the minimum amount is defined.

The content of the active ingredient in the composition may be appropriately determined according to the form of the composition. For example, the content of the active ingredient based on solid content of the composition can be 0.1% or more, preferably 0.3% or more, more preferably 0.6% or more, further preferably 1% or more. The maximum amount of the active ingredient based on solid content may be 50% or less, 30% or less, 20% or less, 10% or less, or 5% or less, no matter how the minimum amount is defined. For the present invention, % means mass percent unless otherwise stated.

When the composition is a liquid, the content of the active ingredient can be, for example, 0.01% or more, preferably 0.03% or more, more preferably 0.06% or more, further preferably 0.1% or more. The maximum content of the active ingredient when the composition is a liquid may be 5% or less, 3% or less, 2% or less, 1% or less, or 0.5% or less, no matter how the minimum content is defined.

### (Other ingredients and additives)

The composition of the present invention may contain other active ingredients or nutritional components that are acceptable as food or pharmaceutical. Examples of such ingredients include lipids (e.g., milk fat, vegetable fats, fats containing medium-chain fatty acids), proteins (e.g., milk proteins, milk protein concentrate (MPC), whey protein concentrate (WPC), whey protein isolate (WPI), α-lactalbumin (α-La), β-lactoglobulin (β-Lg), heat-denatured whey proteins and enzyme-treated whey proteins, amino acids (e.g., lysine, arginine, glycine, alanine, glutamic acid, leucine, isoleucine, and valine), carbohydrates other than human milk oligosaccharides and constituent sugars of human milk oligosaccharides (e.g., glucose, sucrose, fructose, maltose, trehalose, erythritol, maltitol, palatinose, xylitol, and dextrin), electrolytes (e.g., sodium, potassium, calcium, and magnesium salts), vitamins (e.g., vitamin A, vitamin B1, vitamin B2, vitamin B6, vitamin B12, vitamin C, vitamin D, vitamin E, vitamin K, biotin, folic acid, pantothenic acid, and nicotinic acids), minerals (e.g., copper, zinc, iron, cobalt, and manganese), antibiotics, dietary fibers, and so forth.

The composition of the present invention may contain prebiotics other than human milk oligosaccharides. Prebiotics are indigestible food ingredients that selectively alter growth and activity of certain bacteria in the large intestine, thereby favorably affecting the host and improving the host's health. One or more types of prebiotics other than human milk oligosaccharides may be used in the composition.

Prebiotics other than human milk oligosaccharides are not particularly limited so long as they do not interfere with the effect of the active ingredient contained in the composition. Examples of prebiotics other than human milk oligosaccharides include galactooligosaccharides, fructooligosaccharides, xylooligosaccharides, isomaltooligosaccharides, raffinose, lactulose, lactosucrose, soy oligosaccharides, coffee oligosaccharides, dietary fibers, and gluconic acid.

The composition may also further contain an additive acceptable for foods or pharmaceuticals. Examples of such an additive include inactive carriers (solid and liquid carriers), excipients, surfactants, binders, disintegrating agents, lubricants, dissolving aids, suspending agents, coating agents, colorants, preservatives, buffering agents, pH adjustors, emulsifiers, stabilizers, sweeteners, antioxidants, perfumes, acidulants, and natural substances. More specific examples include water, other aqueous solvents, pharmaceutically acceptable organic solvents, collagen, polyvinyl alcohol, polyvinylpyrrolidone, carboxyvinyl polymer, sodium alginate, water-soluble dextran, water-soluble dextrin, carboxymethyl starch sodium, pectin, xanthan gum, gum arabic, casein, gelatin, agar, glycerin, propylene glycol, polyethylene glycol, vaseline, paraffin, stearyl alcohol, stearic acid, human serum albumin, mannitol, sorbitol, lactose, sucralose, stevia, aspartame, acesulfame potassium, citric acid, lactic acid, malic acid, tartaric acid, phosphoric acid, acetic acid, fruit juice, vegetable juice, and so forth.

### (Dosage form or shape)

The composition of the present invention in the form of a food may be prepared in an arbitrary form such as solid, liquid, mixture, suspension, powder, granule, paste, jelly, gel, and capsule. The composition of the present invention in the form of a food can be made in such an arbitrary form as dairy product, supplement, confectionery, drink, drinkable preparation, seasoning, processed food, daily dish, and soup. More specifically, the composition of the present invention may be in the form of liquid diet, semi-liquid diet, jelly, gel, powder, special formula powdered milk, special formula liquid milk, powdered and liquid milk for pregnant and nursing women, fermented milk, bar, mousse, chocolate, biscuit, ice cream, fermented milk, lactic acid bacteria beverage, lactic beverage, milk beverage, soft drink, tablet, cheese, bread, biscuit, cracker, pizza crust, food for sick persons, nutritional food, frozen food, processed food, or the like, or may be in a form of granule, powder, paste, thick solution or the like for being mixed in drink or food and then ingested. Granules and powders can be in the form of cubes or sticks (single-dose packets). For the present invention, special formula powdered milk means a product obtained from a product produced by processing a food made from raw milk, cow's milk, special cow's milk, or raw water buffalo's milk or a food made by using them as a main raw material, by adding nutrients necessary for infants and young children, and making the resulting composition into powder as defined in the "Ministerial Ordinance on Milk and Milk products Concerning Compositional Standards, etc. (hereinafter referred to as the "Ministerial Ordinance on Milk, etc.")". For the present invention, special formula liquid milk means a product obtained from a product produced by processing a food made from raw milk, cow's milk, special cow's milk, or raw water buffalo's milk or a food made by using them as a main raw material, by adding nutrients necessary for infants and young children, and making the resulting composition into liquid as defined in the Ministerial Ordinance on Milk, etc.

Special formula powdered milk and special formula liquid milk (these are sometimes collectively referred to as special formula powdered milk etc., and the explanation of the special formula powdered milk etc. may be made for special formula powdered milk as an example, but such an explanation shall also apply to special formula liquid milk) can be for infants, follow-up (as a supplement to weaning food), low birth weight infants, children, adults, expectant mothers, nursing mothers, elderly persons, those with allergies, those with lactose intolerance, and those with congenital metabolic disorders. Preferred examples of special formula powdered milk etc. are those for infants, follow-up, low birth weight infants, and children.

The composition of the present invention as a pharmaceutical can be in an arbitrary dosage form, for example, those suitable for oral administration including solid preparations such as tablet, granule, powder, pill, and capsule and liquid preparations such as solution, suspension, and syrup, gel, aerosol, or the like.

### (Others)

In the manufacture of the composition of the present invention, time of adding the active ingredient can be appropriately chosen. Unless the characteristics of the active ingredient are not markedly degraded, the time of the addition is not particularly limited. For example, the active ingredient can be blended by mixing it with raw materials. Alternatively, the active ingredient can be added at the final stage of the manufacture to produce a composition containing the active ingredient.

The composition of the invention can have an indication describing the purpose of use (intended use), or an indication describing that administration of the composition is recommended for specific subjects.

The composition of the present invention can have an indication describing that, with the composition or the active ingredient, growth of bacteria of the genus *Eggerthella* in the intestinal tract can be controlled, growth of bacteria of the genus *Eggerthella* in the intestinal tract can be suppressed, or a disease or condition that can be ameliorated by controlling (preferably suppressing) growth of bacteria of the genus *Eggerthella* can be treated, the composition can be used as a prebiotic or can be used as a synbiotic (a combination of a probiotic and a prebiotic), any selected from the group consisting of inflammatory bowel disease, multiple sclerosis, rheumatoid arthritis, mood disorder, and asthma can be treated with the composition or the active ingredient, the composition is suitable to be administered to a patient with colitis aggravated by activation of helper T17 cells, or the composition is suitable to be administered to a patient with heart failure or arrhythmia who is being administered digoxin.

The composition of the present invention can have an indication describing that, with the composition or the active ingredient, growth of bacteria of the genus *Oscillibacter* in the intestinal tract can be controlled, growth of bacteria of the genus *Oscillibacter* in the intestinal tract can be suppressed, or a disease or condition that can be ameliorated by controlling (preferably suppressing) growth of bacteria of the genus *Oscillibacter* can be treated, the composition can be used as a prebiotic or can be used as a synbiotic (a combination of a probiotic and a prebiotic), or any selected from the group consisting of chronic kidney disease, mood disorder, and inflammatory bowel disease can be treated with the composition or the active ingredient.

The composition of the present invention can have an indication describing that, with the composition or the active ingredient, growth of bacteria of the genus *Holdemania* in the intestinal tract can be controlled, growth of bacteria of the genus *Holdemania* in the intestinal tract can be suppressed, or a disease or condition that can be ameliorated by controlling (preferably suppressing) growth of bacteria of the genus *Holdemania* can be treated, the composition can be used as a prebiotic or can be used as a synbiotic (a combination of a probiotic and a prebiotic), or any selected from the group consisting of mood disorder and Parkinson's disease can be treated with the composition or the active ingredient.

The composition of the present invention can have an indication describing that, with the composition or the active ingredient, growth of bacteria of the genus *Parasutterella* in the intestinal tract can be controlled, growth of bacteria of the genus *Parasutterella* in the intestinal tract can be suppressed, or a disease or condition that can be ameliorated by controlling (preferably suppressing) growth of bacteria of the genus *Parasutterella* can be treated, the composition can be used as a prebiotic or can be used as a synbiotic (a combination of a probiotic and a prebiotic), or any selected from the group consisting of mood disorder, irritable bowel syndrome, and liver dysfunction can be treated with the composition or the active ingredient.

The indication may be a direct or indirect indication. Examples of the direct indication include descriptions on tangible articles such as the product itself, package, container, label, and tag, and examples of the indirect indication includes advertising and campaign activities using such places or means as web site, shop, pamphlet, exhibition, seminar such as media seminar, book, newspaper, magazine, television, radio, postal matter, E-mail, and sound.

Hereafter, the present invention will be more specifically explained with reference to examples. However, the technical scope of the present invention is not limited by these examples.

### Examples

### 1. Example concerning bacteria of the genus Eggerthella

### [Preparation of sugar solutions]

2'-FL (Jennewein Biotechnologie GmbH), 3'-SL (Carbosynth), 6'-SL (Tokyo Kasei Kogyo, #S0886), lactose (Fujifilm Wako Pure Chemicals Corporation, #128-00095), and fucose (Fujifilm Wako Pure Chemicals Corporation, #068-05323) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 70 µL was added to 7 mL of GAM semisolid medium without dextrose (Nissui, #05460) for glycolysis test and incubated at 37°C for 24 hours under anaerobic conditions. Each fecal culture was added to 6 wells in total of a 96-well deep well plate in a volume of 900 µL each. To the 6 wells, the sugar solutions (5 types) and sterilized water were added, respectively, in a volume of 100 µL each (final concentrations of the sugar solutions, 1 mass %). After incubation at 37°C for 48 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

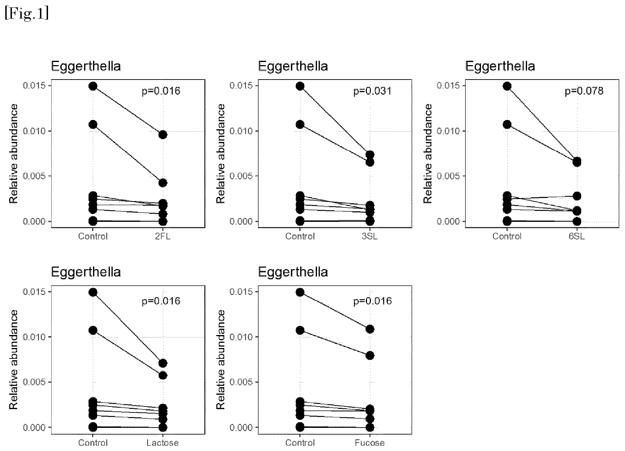

The results are shown in Fig. 1. Compared with the sterile water group as the control, the occupancy rate of bacteria of the genus *Eggerthella* significantly decreased or tended to decrease in the groups in which 2'-FL, 3'-SL, 6'-SL, fucose and lactose were added. These results suggested that 2'-FL, 3'-SL, 6'-SL, fucose, and lactose suppress the growth of bacteria of the genus *Eggerthella* in the human intestinal tract. By the way, bacteria of the genus *Bifidobacterium* were detected in all the thawed fecal solutions.

In this example, a growth-suppressing effect of lactose for bacteria of the genus *Eggerthella* was confirmed. HMOs contain lactose as a constituent sugar, and HMOs are thought to generate lactose in the human digestive tract after ingestion (Non-patent document 13). Therefore, it is considered that a growth-suppressing effect for bacteria of the genus *Eggerthella* can be obtained with HMOs.

In this example, a growth-suppressing effect of fucose for bacteria of the genus *Eggerthella* was confirmed. HMOs containing fucose as a constituent sugar are thought to generate fucose in the human digestive tract after ingestion (Non-patent documents 13 and 14). Therefore, it is considered that a growth-suppressing effect for bacteria of the genus *Eggerthella* can be obtained with HMOs containing fucose as a constituent sugar (fucosylated HMOs and HMOs containing fucose).

In this example, a growth-suppressing effect for bacteria of the genus *Eggerthella* was confirmed for all of 2'-FL, 3'-SL, and 6'-SL. From these results, it is considered that a growth-suppressing effect for bacteria of the genus *Eggerthella* can be obtained with fucosylated HMOs and sialylated HMOs.

### 2. Examples concerning bacteria of the genus Oscillibacter

### <Example 1>

### [Preparation of sugar solutions]

2'-FL (Jennewein Biotechnologie GmbH) and lactose (Fujifilm Wako Pure Chemicals Corporation, #128-00095) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 60 µL was added to 6 mL of GAM semisolid medium without dextrose (Nissui, #05460) for glycolysis test and incubated at 37°C for 24 hours under anaerobic conditions. Each fecal culture was added to 5 wells in total of a 96-well deep well plate in a volume of 900 µL each. To the 5 wells, the sugar solutions (4 types) and sterilized water were added, respectively, in a volume of 100 µL each (final concentrations of the sugar solutions, 1 mass %). After incubation at 37°C for 24 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

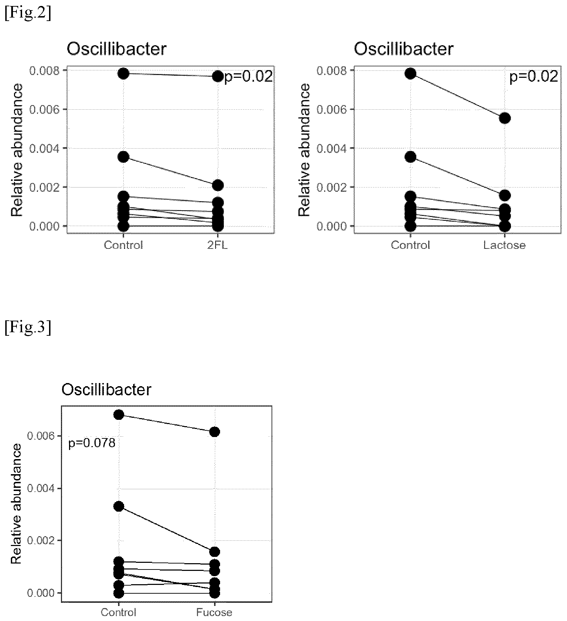

The results are shown in Fig. 2. Compared with the sterile water group as the control, the occupancy rate of bacteria of the genus *Oscillibacter* significantly decreased in the 2'-FL and lactose-added groups. These results suggested that 2'-FL and lactose suppress growth of bacteria of the genus *Oscillibacter* in the human intestinal tract.
By the way, bacteria of the genus *Bifidobacterium* were detected in all the thawed fecal solutions.

### <Example 2>

### [Preparation of sugar solution]

Fucose (Fujifilm Wako Pure Chemicals Corporation # 068-05323) was dissolved in sterile water at 10 mass %, and then the solution was sterilized with a 0.22 µm filter (Merck Millipore, #0369).

### [Effect of human milk oligosaccharide on intestinal bacterial microbiota in human fecal fermentation system]

Frozen fecal solutions provided by 8 healthy adults were thawed on a 37°C water bath. Each thawed fecal solution in a volume of 30 µL was added to 3 mL of GAM semisolid medium without dextrose (Nissui, #05460) for glycolysis test and incubated at 37°C for 24 hours under anaerobic conditions. Each fecal culture was added to 2 wells in total of a 96-well deep well plate in a volume of 900 µL each. To the 2 wells, the sugar solution and sterilized water were added, respectively, in a volume of 100 µL each (final concentration of the sugar solution, 1 mass %). After incubation at 37°C for 48 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

The results are shown in Figure 3. Compared with the sterile water group as the control, the occupancy rate of bacteria of the genus *Oscillibacter* tended to decrease in the fucose-added group. These results suggested that fucose suppresses growth of bacteria of the genus *Oscillibacter* in the human intestinal tract. By the way, bacteria of the genus *Bifidobacterium* were detected in all the thawed fecal solutions.

### <Subsummary>

In this example, a growth-suppressing effect of lactose for bacteria of the genus *Oscillibacter* was confirmed. HMOs contain lactose as a constituent sugar, and are thought to generate lactose in the human digestive tract after ingestion (Non-patent document 13). Therefore, it is considered that a growth-suppressing effect for bacteria of the genus *Oscillibacter* can be obtained with HMOs.

In this example, a growth-suppressing effect of fucose for bacteria of the genus *Oscillibacter* was confirmed. HMOs containing fucose as a constituent sugar are thought to generate fucose in the human digestive tract after ingestion (Non-patent documents 13 and 14). Therefore, it is considered that a growth-suppressing effect for bacteria of the genus *Oscillibacter* can be obtained with HMOs containing fucose as a constituent sugar (fucosylated HMOs and HMOs containing fucose).

In this example, a growth-suppressing effect of 2'-FL for bacteria of the genus *Oscillibacter* was confirmed. Therefore, it is considered that a growth-suppressing effect for bacteria of the genus *Oscillibacter* can be obtained with fucosylated HMOs.

### 3. Example concerning bacteria of the genus Holdemania:

### [Preparation of sugar solutions]

2'-FL (Jennewein Biotechnologie GmbH), 3'-SL (Carbosynth), 6'-SL (Tokyo Kasei Kogyo, #S0886), lactose (Fujifilm Wako Pure Chemicals Corporation, #128-00095), and Neu5Ac (Fujifilm Wako Pure Chemicals Corporation, #011-26173) were dissolved in sterile water at 10 mass %, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 70 µL was added to 7 mL of GAM semisolid medium without dextrose (Nissui, #05460) for glycolysis test and incubated at 37°C for 24 hours under anaerobic conditions. Each fecal culture was added to 6 wells in total of a 96-well deep well plate in a volume of 900 µL each. To the 6 wells, the sugar solutions (5 types) and sterilized water were added, respectively, in a volume of 100 µL each (final concentrations of the sugar solutions, 1 mass %). After 48 hours of incubation at 37°C under anaerobic conditions, DNA was extracted with Maxwell (manufacturer's name, Promega), and amplicon sequencing of the V3-V4 region was performed with MiSeq (manufacturer's name, Illumina).

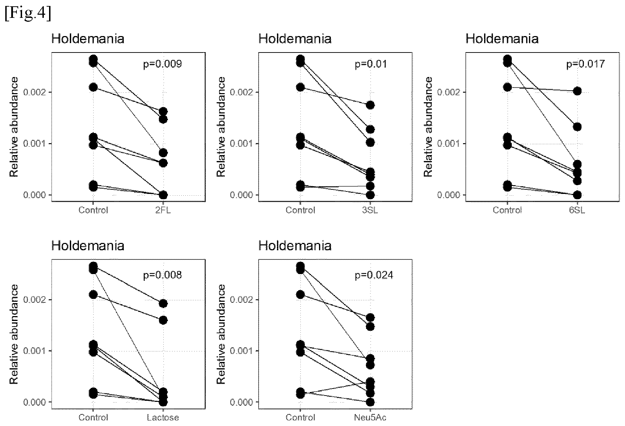

The results are shown in Fig. 4. Compared with the sterile water group as the control, the occupancy rate of bacteria of the genus *Holdemania* significantly decreased in the groups for which 2'-FL, 3'-SL, 6'-SL, lactose, and Neu5Ac were added. These results suggested that 2'-FL, 3'-SL, 6'-SL, lactose, and Neu5Ac suppress growth of bacteria of the genus *Holdemania* in the human intestinal tract. By the way, bacteria of the genus *Bifidobacterium* were detected in all the thawed fecal solutions.

In this example, a growth-suppressing effect of lactose for bacteria of the genus *Holdemania* was confirmed. HMOs contain lactose as a constituent sugar, and HMOs are thought to generate lactose in the human digestive tract after ingestion (Non-patent document 12). Therefore, it is considered that a growth-suppressing effect for bacteria of the genus *Holdemania* can be obtained with HMOs.

In this example, a growth-suppressing effect of Neu5Ac for bacteria of the genus *Holdemania* was confirmed. HMOs containing Neu5Ac as a constituent sugar are thought to generate Neu5Ac in the human digestive tract after ingestion (Non-patent documents 13, 15, and 16). Therefore, it is considered that a growth-suppressing effect for bacteria of the genus *Holdemania* can be obtained with HMOs containing Neu5Ac as a constituent sugar (sialylated HMOs and HMOs containing sialic acid).

In this example, a growth-suppressing effect for bacteria of the genus *Holdemania* was confirmed for all of 2'-FL, 3'-SL, and 6'-SL. From these results, it is considered that a growth-suppressing effect for bacteria of the genus *Holdemania* can be obtained with fucosylated HMOs and sialylated HMOs.

### 4. Examples concerning bacteria of the genus Parasutterella

### <Example 1>

### [Preparation of sugar solutions]

2'-FL (Jennewein Biotechnologie GmbH.), 3'-SL (Carbosynth), lactose (Fujifilm Wako Pure Chemicals Corporation, #128-00095), and Neu5Ac (Fujifilm Wako Pure Chemicals Corporation, #011-26173) were dissolved in sterile water at 10 mass percent, and then the solutions were sterilized with a 0.22 µm filter (Merck Millipore, #0369).

### [Effect of human milk oligosaccharides on intestinal bacterial microbiota in human fecal fermentation system]

Frozen fecal solutions provided by 8 healthy adults were thawed on a 37°C water bath. Each thawed fecal solution in a volume of 60 µL was added to 6 mL of GAM semisolid medium without dextrose (Nissui, #05460) for glycolysis test and incubated at 37°C for 24 hours under anaerobic conditions. Each fecal culture was added to 5 wells in total of a 96-well deep well plate in a volume of 900 µL each. To the 5 wells, the sugar solutions (4 types) and sterilized water were added, respectively, in a volume of 100 µL each (final concentrations of the sugar solutions, 1 mass %). After 24 hours of incubation at 37°C under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

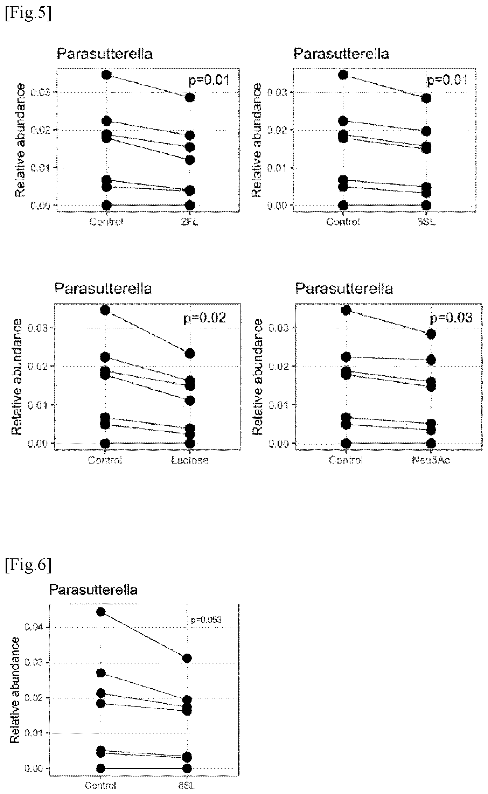

The results are shown in Fig. 5. Compared with the sterile water group as the control, the occupancy rate of bacteria of the genus *Parasutterella* significantly decreased in the groups in which 2'-FL, 3'-SL, lactose, and Neu5Ac were added. These results suggested that 2'-FL, 3'-SL, lactose, and Neu5Ac suppress growth of bacteria of the genus *Parasutterella* in the human intestinal tract. By the way, bacteria of the genus *Bifidobacterium* were detected in all the thawed fecal solutions.

### <Example 2>

### [Preparation of sugar solution]

6'-SL (Tokyo Kasei Kogyo #S0886) was dissolved in sterile water at 10 mass %, and then the solution was sterilized with a 0.22 µm filter (Merck Millipore, #0369).

### [Effect of human milk oligosaccharide on intestinal bacterial microbiota in human fecal fermentation system]

Frozen fecal solutions provided by 8 healthy adults were thawed on a 37°C water bath. Each thawed fecal solution in a volume of 30 µL was added to 3 mL of GAM semisolid medium without dextrose (Nissui, #05460) for glycolysis test and incubated at 37°C for 24 hours under anaerobic conditions. Each fecal culture was added to 2 wells in total of a 96-well deep well plate in a volume of 900 µL each. To the 2 wells, the sugar solution and sterilized water were added, respectively, in a volume of 100 µL each (final concentration of the sugar solution, 1 mass %). After incubation at 37°C for 24 hours under anaerobic conditions, DNA was extracted with Maxwell (manufacturer's name, Promega), and amplicon sequencing of the V3-V4 region was performed with MiSeq (manufacturer's name, Illumina). The resulting fastq files were analyzed with QIIME2 (https://qiime2.org/) to calculate the occupancy rate of each type of intestinal bacteria.

The results are shown in Fig. 6. Compared with the distilled water group as the control, the occupancy rate of bacteria of the genus *Parasutterella* tended to decrease in the 6'-SL-added group.

This result suggested that 6'-SL suppresses growth of bacteria of the genus *Parasutterella* in the human intestinal tract. By the way, bacteria of the genus *Bifidobacterium* were detected in all the thawed fecal solutions.

### <Subsummary>

In this example, a growth-suppressing effect of lactose for bacteria of the genus *Parasutterella* was confirmed. HMOs contain lactose as a constituent sugar, and HMOs are thought to generate lactose in the human digestive tract after ingestion (Non-patent document 13). Therefore, it is considered that a growth-suppressing effect for bacteria of the genus *Parasutterella* can be obtained with HMOs.

In this example, a growth-suppressing effect of Neu5Ac for bacteria of the genus *Parasutterella* was confirmed. HMOs containing Neu5Ac as a constituent sugar are thought to generate Neu5Ac in the human digestive tract after ingestion (Non-patent documents 13, 15, and 16). Therefore, it is considered that growth-suppressing effect for bacteria of the genus *Parasutterella* can be obtained with HMOs containing Neu5Ac as a constituent sugar (sialylated HMOs and HMOs containing sialic acid).

In this example, a growth-suppressing effect for bacteria of the genus *Parasutterella* was confirmed for all of 2'-FL, 3'-SL, and 6'-SL. From these results, it is considered that a growth-suppressing effect for bacteria of the genus *Parasutterella* can be obtained with fucosylated HMOs and sialylated HMOs.

### 5. References cited in the examples

Non-patent document 13: Masi AC, Stewart CJ. Untangling human milk oligosaccharides and infant gut microbiome. iScience. 2021;25(1):103542. Published 2021 Dec 1. doi:10.1016/j.isci.2021.103542
Non-patent document 14: Katayama T, Sakuma A, Kimura T, Makimura Y, Hiratake J, Sakata K, Yamanoi T, Kumagai H, Yamamoto K. Molecular cloning and characterization of Bifidobacterium bifidum 1,2-alpha-L-fucosidase (AfcA), a novel inverting glycosidase (glycoside hydrolase family 95). J Bacteriol. 2004 Aug;186(15):4885-93. doi: 10.1128/JB.186.15.4885-4893.2004. PMID: 15262925; PMCID: PMC451662
Non-patent document 15: Nishiyama K, Yamamoto Y, Sugiyama M, et al. Bifidobacterium bifidum Extracellular Sialidase Enhances Adhesion to the Mucosal Surface and Supports Carbohydrate Assimilation. mBio. 2017;8(5):e00928-17. Published 2017 Oct 3. doi:10.1128/mBio.00928-17
Non-patent document 16: Kiyohara M, Tanigawa K, Chaiwangsri T, Katayama T, Ashida H, Yamamoto K. An exo-alpha-sialidase from bifidobacteria involved in the degradation of sialyloligosaccharides in human milk and intestinal glycoconjugates. Glycobiology. 2011;21(4):437-447. doi: 10.1093/glycob/cwq 175

## Claims

1. A composition for controlling growth of any bacteria selected from the group consisting bacteria of the genus *Eggerthella*, bacteria of the genus *Oscillibacter*, bacteria of the genus *Holdemania*, and bacteria of the genus *Parasutterella* in the intestinal bacterial microbiota, which contains any selected from the group consisting of a human milk oligosaccharide and a constituent sugar of a human milk oligosaccharide.

2. The composition according to claim 1, which contains any selected from the group consisting of a fucosylated human milk oligosaccharide, a sialylated human milk oligosaccharide, a human milk oligosaccharide containing lactose as a constituent sugar, a human milk oligosaccharide containing fucose as a constituent sugar, lactose, and fucose, and is for controlling growth of bacteria of the genus *Eggerthella.*

3. The composition according to claim 2, which contains any selected from the group consisting of 2'-fucosyllactose, 3'-sialyllactose, 6'-sialyllactose, lactose, and fucose.

4. The composition according to claim 1, which contains any selected from the group consisting of a fucosylated human milk oligosaccharide, a human milk oligosaccharide containing lactose as a constituent sugar, a human milk oligosaccharide containing fucose as a constituent sugar, lactose, and fucose, and is for controlling growth of any bacteria selected from the group consisting of bacteria of the genus *Eggerthella* and bacteria of the genus *Oscillibacter.*

5. The composition according to claim 4, which contains any selected from the group consisting of 2'-fucosyllactose, lactose, and fucose.

6. The composition according to claim 1, which contains any selected from the group consisting of a fucosylated human milk oligosaccharide, a sialylated human milk oligosaccharide, a human milk oligosaccharide containing lactose as a component sugar, a human milk oligosaccharide containing sialic acid as a constituent sugar, lactose, and sialic acid, and is for controlling growth of any bacteria selected from the group consisting of bacteria of the genus *Holdemania* and bacteria of the genus *Parasutterella.*

7. The composition according to claim 6, which contains any selected from the group consisting of 2'-fucosyllactose, 3'-sialyllactose, 6'-sialyllactose, lactose, and N-acetylneuraminic acid.

8. The composition according to any one of claims 1 to 7, which is for use as a prebiotic or synbiotic.

9. The composition according to any one of claims 1 to 7, which is for treating mood disorder.

10. A composition for treating a disease or condition that can be ameliorated by controlling growth of any bacteria selected from the group consisting of bacteria of the genus *Eggerthella*, bacteria of the genus *Oscillibacter*, bacteria of the genus *Holdemania,* and bacteria of the genus *Parasutterella* in the intestinal tract, which contains any selected from the group consisting of a human milk oligosaccharide and a constituent sugar of a human milk oligosaccharide.

11. A composition for treating mood disorder, which contains any selected from the group consisting of a human milk oligosaccharide and a constituent sugar of a human milk oligosaccharide.

12. The composition according to any one of claims 1 to 7, wherein the control of growth is suppression of growth.
